# EUROPEAN PATENT APPLICATION

(11) **EP 2 223 647 A1**
(43) Date of publication of application: **01.09.2010**
(21) Application number: 10154706.5
(22) Date of filing: 25.02.2010
(51) Int. Cl.: A61B 1/31, A61M 25/01, G02B 23/24

(54) **Actuator for moving body in tube, method for controlling the same, and endoscope**

(30) Priority: 26.02.2009 JP 2009044572; 06.01.2010 JP 2010001364
(71) Applicant: FUJIFILM Corporation, Tokyo 106-8620 (JP)
(72) Inventor: Yamakawa, Shinichi, Saitama 331-9624 (JP); Ashida, Tsuyoshi, Saitama 331-9624 (JP); Nakamura, Takayuki, Saitama 331-9624 (JP); Miyako, Kuniaki, Saitama 331-9624 (JP); Morimoto, Yuya, Saitama 331-9624 (JP)
(74) Representative: Klunker . Schmitt-Nilson . Hirsch

(57) **Abstract**

An aspect of the actuator for the moving body includes: a first expansion/contraction member (22, 94) which includes a first portion that fills between the moving body in tube and the tube wall at the time of being expanded to come into contact with the tube wall, and a second portion that is brought into contact with the tube wall to generate propulsive force, and a part of which is fixed to the moving body in tube; a drive device (34, 36, 76, 78) which drives the first expansion/contraction member (22, 94); and a control section (30, 32, 72, 74, 80) which controls the first expansion/contraction member and the drive device, and is **characterized in that** the control section performs control so that the first portion of the first expansion/contraction member is made to become the second portion by the drive by the drive device so as to change the relative position between the moving body in tube and the tube wall.

## Description

### BACKGROUND OF THE INVENTION

### Field of the Invention

The present invention relates to an actuator for a moving body in tube, a method for controlling the actuator, and an endoscope. More particularly, the present invention relates to a technique to effect movement in a tube by transmitting propulsive force to the tube wall.

### Description of the Related Art

The insertion of an endoscope into the large intestine is very difficult because the large intestine is wound in the body and because a part of the large intestine is not fixed to a body cavity. Therefore, much experience is required to acquire the insertion technique. When the insertion technique is insufficient, it causes significant pain to the patient.

Portions of the large intestine, in which portions the endoscope insertion is said to be difficult, are the sigmoid colon and the transverse colon. Unlike other colons, the sigmoid colon and the transverse colon are not fixed in the body cavity. Therefore, they can take an arbitrary shape in the body cavity in the range of their own length, and can be deformed in the body cavity by the contact force at the time of endoscope insertion.

When the endoscope is inserted into the large intestine, it is important to straighten the sigmoid colon and the transverse colon in order to reduce the contact with the intestinal tract at the time of insertion as much as possible. Many straightening techniques have hitherto been proposed. Also, there have been proposed several insertion assisting tools used to draw the curved intestinal tract so as to reduce the degree of curvature of the intestinal tract.

For example, in Japanese Patent Application Laid-Open No. 11-9545 and Japanese Patent Application Laid-Open No. 2006-223895, there is disclosed a technique in which four expansible/contractible variable tubes are spirally wound around the outer peripheral surface of a flexible tube section, and in which the intestinal tract is drawn in such a manner that the four variable tubes are successively expanded and contracted by changing the pressure in each of the variable tubes so that the outer peripheral surface of the skin of the four variable tubes is successively expanded and contracted to enable the expanded section to be moved from the distal end side to the hand side.

### SUMMARY OF THE INVENTION

However, when the plurality of variable tubes are only vertically moved, almost no effect of moving the contact surface of the tube is obtained. The effect of the drawing can be obtained only when the fold of the intestinal tract is made to efficiently enter the groove between the expanded tubes. However, almost no fold exists in the sigmoid colon. Further, as the intestinal tract is straightened in the process of being drawn, the projection amount of the fold is reduced, so that the effect of the drawing is significantly reduced. Therefore, it is difficult that one balloon is made to generate engagement force so as to engage with the intestinal wall and is also made to generate propulsive force so as to move relatively to the intestinal wall.

The present invention has been made in view of the above described circumstances. An object of the present invention is to provide an actuator for a moving body in tube, which is capable of moving the moving body in tube by surely drawing the tube wall, and to provide a method for controlling the actuator, and an endoscope.

In order to achieve the above described objects, an actuator for a moving body in tube, according to the present invention, is characterized by including: a first expansion/contraction member which includes a first portion that fills between the moving body in tube and a tube wall at the time of being expanded to come into contact with the tube wall, and a second portion that is brought into contact with the tube wall to generate propulsive force, and a part of which is fixed to the moving body in tube; a drive device which drives the first expansion/contraction member; and a control section which controls the first expansion/contraction member and the drive device, and is featured in that the control section performs control so that the first portion of the first expansion/contraction member is made to become the second portion by the drive by the drive device so as to change the relative position between the moving body in tube and the tube wall.

According to the present invention, the moving body in tube can be moved because the control is performed such that the first portion of the first expansion/contraction member is made to become the second portion by the drive by the drive device so as to change the relative position between the moving body in tube and the tube wall.

An aspect of the present invention is characterized in that the drive device is a second expansion/contraction member which is arranged side by side with the first expansion/contraction member in the direction in which the moving body in tube is moved inside the tube, and which is fixed to the moving body in tube, and in that the control section performs control so that after the first expansion/contraction member is expanded to engage with the tube wall, the second expansion/contraction member is expanded to press the first expansion/contraction member.

According to the present invention, after the first expansion/contraction member is expanded to engage with the tube wall, the second expansion/contraction member is expanded to press the first expansion/contraction member, and hence the moving body in tube can be surely moved.

An aspect of the present invention is characterized in that the control section performs control so that the first expansion/contraction member is pressed by the second expansion/contraction member to thereby cause the tube wall to be drawn.

According to the aspect of the present invention, the first expansion/contraction member is pressed by the second expansion/contraction member to thereby cause the tube wall to be drawn, and hence the relative position between the moving body in tube and the tube wall can be surely changed.

An aspect of the present invention is characterized in that the control section performs control so that the tube wall is drawn by feeding the surface of the first expansion/contraction member out.

According to the aspect of the present invention, the control is performed so that the tube wall is drawn by the feeding of the surface of the first expansion/contraction member. Thereby, the propulsive force is generated on the tube wall, so that the moving body in tube can be moved by more surely drawing the tube wall.

An aspect of the present invention is characterized in that the first expansion/contraction member, in the state of being expanded to engage with the tube wall, covers at least a part of the contracted second expansion/contraction member.

According to the aspect of the present invention, the first expansion/contraction member, in the state of being expanded to engage with the tube wall, covers at least a part of the contracted second expansion/contraction member. Thereby, the first expansion/contraction member can be surely pressed by the expansion of the second expansion/contraction member.

An aspect of the present invention is characterized in that the second expansion/contraction member has directivity in the direction of deformation.

According to the aspect of the present invention, the second expansion/contraction member has directivity in the direction of deformation, and hence the drawn amount of the tube wall can be increased.

An aspect of the present invention is characterized in that at least one of the first expansion/contraction member and the second expansion/contraction members is a balloon.

An aspect of the present invention is characterized by including a third expansion/contraction member which is provided at the moving body in tube so as to be arranged side by side with the first expansion/contraction member and the second expansion/contraction member in the direction in which the moving body in tube is moved inside the tube, and which is arranged on the side opposite to the second expansion/contraction member across the first expansion/contraction member.

According to the aspect of the present invention, the third expansion/contraction member, which is arranged on the side opposite to the second expansion/contraction member across the first expansion/contraction member, is provided, and hence it is possible to arbitrarily select the direction in which the moving body in tube is moved.

An aspect of the present invention is characterized in that the control section performs control so that before the first expansion/contraction member is expanded to engage with the tube wall, the third expansion/contraction member is expanded to press the first expansion/contraction member.

According to the aspect of the present invention, the control is performed such that before the first expansion/contraction member is expanded to engage with the tube wall, the third expansion/contraction member is expanded to press the first expansion/contraction member. Thus, it is possible to increase the drawn amount of the tube wall.

An aspect of the present invention is characterized in that the first expansion/contraction member, in the state of being expanded to engage with the tube wall, covers at least a part of the contracted third expansion/contraction member.

According to the aspect of the present invention, the first expansion/contraction member, in the state of being expanded to engage with the tube wall, covers at least a part of the contracted third expansion/contraction member. Thus, the first expansion/contraction member can be more surely pressed by the expansion of the third expansion/contraction member.

An aspect of the present invention is characterized in that the third expansion/contraction member has directivity in the direction of deformation.

According to the aspect of the present invention, the third expansion/contraction member has directivity in the direction of deformation, and hence the drawn amount of the tube wall can be more surely increased.

An aspect of the present invention is characterized in that the first expansion/contraction member and the second expansion/contraction member are arranged in this order from the back side in the direction in which the moving body in tube is moved inside the tube.

According to the aspect of the present invention, the first expansion/contraction member and the second expansion/contraction member are arranged in this order from the back side in the direction in which the moving body in tube is moved inside the tube. Thus, the moving body in tube can be moved forward in its moving direction inside the tube.

An aspect of the present invention is characterized in that a plurality of combinations of the first expansion/contraction member and the second expansion/contraction member are provided at the moving body in tube.

According to the aspect of the present invention, the moving body in tube can be surely moved by drawing the tube wall at a plurality of places inside the tube.

An aspect of the present invention is characterized in that the control section performs control so that the internal pressure of the second expansion/contraction member becomes equal to or greater than the internal pressure of the first expansion/contraction member.

According to the aspect of the present invention, the second expansion/contraction member can be expanded to a sufficient size without being pressed by the first expansion/contraction member, and reduction of propulsive force can be prevented.

An aspect of the present invention is characterized in that a holding member which engages with the tube wall to hold the position of the moving body in tube is provided at the moving body in tube.

According to the aspect of the present invention, the position of the moving body in tube can be held by the holding member, even when the first expansion/contraction member and the second expansion/contraction member do not engage with the tube wall.

In order to achieve the above described objects, an endoscope according to the present invention is featured by including one of the above described actuators for the moving body in tube.

In order to achieve the above described objects, a control method of an actuator for a moving body in tube, according to the present invention, is featured by performing control to drive a first expansion/contraction member which includes a first portion that fills between the moving body in tube and a tube wall at the time of being expanded to come into contact with the tube wall, and a second portion that is brought into contact with the tube wall to generate propulsive force, and a part of which is fixed to the moving body in tube, so that the first portion of the first expansion/contraction member becomes the second portion so as to change the relative position between the moving body in tube and the tube wall.

According to the present invention, the moving body in tube can be moved by surely drawing the tube wall.

### BRIEF DESCRIPTION OF THE DRAWINGS

Fig. 1 is a view showing a configuration of an electronic endoscope;
Fig. 2 is an enlarged sectional view showing a distal end section of an insertion section of example 1 according to a first embodiment;
Fig. 3 is a block diagram of a balloon control apparatus which controls the pressure of a drive balloon and an engagement balloon;
Fig. 4 is a time chart of a propulsion operation in example 1 according to the first embodiment;
Figs. 5A to 5E are schematic sectional views showing states of expansion and contraction of the respective balloons in example 1 according to the first embodiment in correspondence with the time chart of the propulsion operation shown in Fig. 4;
Fig. 6 is an enlarged sectional view showing a distal end section of an insertion section in example 2 according to the first embodiment;
Figs. 7A to 7E are schematic sectional views showing states of expansion and contraction of the respective balloons in example 2 according to the first embodiment in correspondence with the time chart of the propulsion operation shown in Fig. 4;
Fig. 8 is an enlarged sectional view showing a distal end section 10a of an insertion section 10 according to a second embodiment;
Fig. 9 is a time chart at the time when a forward movement operation is performed by using three balloons in the second embodiment;
Figs. 10A to 10F are schematic sectional views showing states of expansion and contraction of the respective balloons in correspondence with the time chart shown in Fig. 9;
Fig. 11 is a time chart at the time when a backward movement operation is performed by using two balloons in the second embodiment;
Figs. 12A to 12E are schematic sectional views showing states of expansion and contraction of the respective balloons in correspondence with the time chart of the forward movement operation shown in Fig. 11;
Fig. 13 is a time chart at the time when a backward movement operation is performed by using three balloons in the second embodiment;
Figs. 14A to 14F are schematic sectional views showing states of expansion and contraction of the respective balloons in correspondence with the time chart shown in Fig. 13;
Fig. 15 is an enlarged sectional view showing a distal end section of an insertion section according to a third embodiment;
Fig. 16 is a block diagram of a balloon control apparatus which controls the pressure of a propulsion balloon and a holding balloon;
Fig. 17 is a time chart of a propulsion operation in the third embodiment;
Figs. 18A to 18F are schematic sectional views showing states of expansion and contraction of the respective balloons in the third embodiment in correspondence with the time chart shown in Fig. 17;
Fig. 19 is an enlarged sectional view showing a distal end section of an insertion section according to a fourth embodiment;
Fig. 20 is a block diagram of a balloon control apparatus which controls the pressure of a propulsion balloon and a holding balloon;
Fig. 21 is a time chart of a propulsion operation in the fourth embodiment;
Figs. 22A to 22F are schematic sectional views showing states of expansion and contraction of the respective balloons in the fourth embodiment in correspondence with the time chart shown in Fig. 21;
Fig. 23 is a view showing an example of application to a moving apparatus for an endoscope; and
Fig. 24 is an explanatory view showing one example of the relationship between the internal pressure of each balloon and propulsive force.

### DETAILED DESCRIPTION OF THE PREFERRED EMBODIMENT(S)

In the following, preferred embodiments according to the present invention will be described in detail with reference to the accompanying drawings.

### [Description of electronic endoscope]

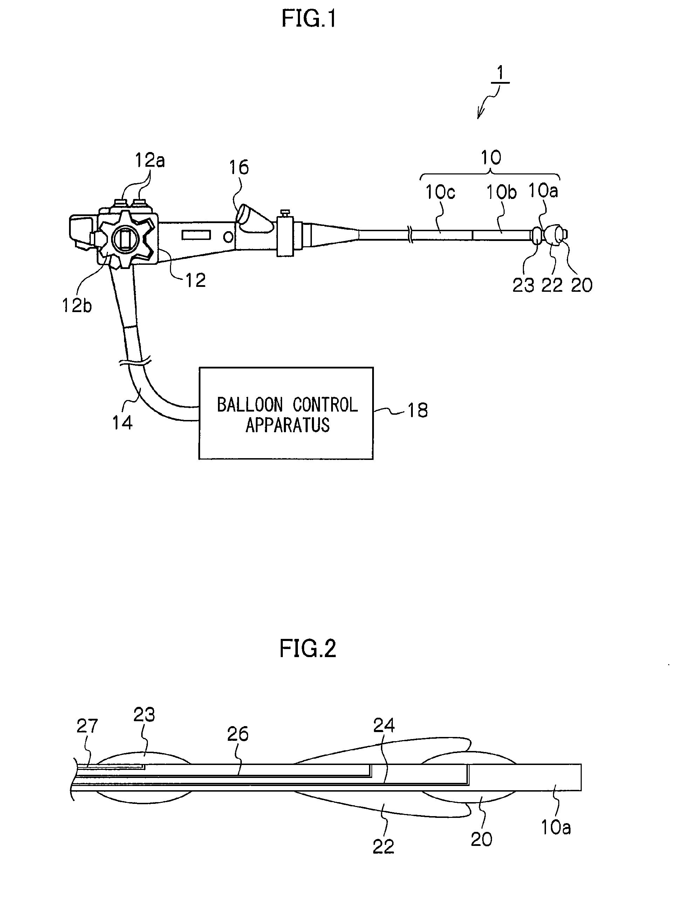

In Fig. 1, an electronic endoscope 1 includes an insertion section 10 which is a moving body in tube that is inserted into a tube of a subject so as to be moved inside the tube, and an operation section 12 which is continuously connected to the proximal end portion of the insertion section 10. An objective lens which captures image light from an observation site inside the subject, and an image pickup element which picks up the image light (both not shown) are incorporated in a distal end section 10a continuously connected to the distal end of the insertion section 10. An image inside the subject, which is captured by the image pickup element, is displayed as an endoscopic image in a monitor of a processor apparatus (both not shown) connected to a code 14.

In the distal end section 10a, there are provided an illumination window for irradiating the observation site with illumination light from a light source apparatus (not shown), a forceps outlet communicating with a forceps opening 16, a nozzle through which wash water and air for removing a stain on an observation window protecting the objective lens are jetted out by the operation of an air and water supply button 12a, and the like.

A bending section 10b formed by connecting a plurality of bending pieces is provided behind the distal end section 10a. When an angle knob 12b provided at the operation section 12 is operated, wires inserted in the insertion section 10 are pushed and pulled, so that the bending section 10b is bent in the up, down, left and right directions. Thereby, the distal end section 10a is directed to a desired direction in the inside of the subject.

A soft section 10c having flexibility is provided behind the bending section 10b.
The soft section 10c has a length of one to several meters so as to enable the distal end section 10a to reach the observation site and so as to allow the operator to maintain a distance from the patient to such an extent that the operation section 12 does not cause any trouble at the time of being grasped and operated by the operator.

To the distal end section 10a, there are attached a drive balloon 20 and an engagement balloon 22 that, as will be described below, are configured as expansion/contraction members arranged and fixed side by side in the advance direction in which the distal end section 10a is moved inside the tube. The drive balloon 20 and the engagement balloon 22 are mainly made of natural rubber such as freely expandable and contractible latex rubber and are connected to a balloon control apparatus 18 which controls the pressure in the balloons. Note that in the distal end section 10a, the drive balloon 20 and the engagement balloon 22 are arranged so as to be adjacent to each other and are formed in the entire circumferential direction of the insertion section 10.
Further, the drive balloon 20 and the engagement balloon 22 may have an axially symmetrical shape which is formed uniformly in the circumferential direction of the insertion section 10. Further, the drive balloon 20 and the engagement balloon 22 may also have an axially asymmetrical shape which is formed non-uniformly in the circumferential direction of the insertion section 10.

Further, the drive balloon 20 and the engagement balloon 22 may be arranged at the bending section 10b and the soft section 10c.

For example, when the inner wall surface of a complicatedly bent tube, such as the large intestine and the small intestine, is observed with the electronic endoscope 1 configured as described above, the insertion section 10 is inserted into the subject in the state where the drive balloon 20 and the engagement balloon 22 are contracted. Then, while the inside of the subject is illuminated by turning on the light source apparatus, an endoscopic image obtained by the image pickup device is observed by the monitor.

When the distal end section 10a reaches the tube, the expansion/contraction of the drive balloon 20 and the engagement balloon 22 are controlled by the balloon control apparatus 18, so that the pressing force is made to act on the inner wall surface of the tube. Thereby, the inner wall surface of the tube is drawn, so that the insertion section 10 is propelled forward or backward in the advance direction of the distal end section 10a relatively to the inner wall surface of the tube.

Note that the flow of the propulsion operation will be described in detail below. In the following description, it is assumed that the operation to propel the distal end section 10a forward in the advance direction is the forward movement operation and that the operation to propel the distal end section 10a backward in the advance direction is the backward movement operation.

### [Description of actuator for moving body in tube]

Next, there will be described an actuator for a moving body in tube.

### [First embodiment]

First, a first embodiment will be described. In the first embodiment, the number of balloons is set to two.

### (Example 1)

### <Configuration of actuator for moving body in tube>

Fig. 2 is an enlarged sectional view showing the distal end section 10a of the insertion section 10 of example 1 according to a first embodiment. As shown in Fig. 2, in example 1, two balloons of the drive balloon 20 and the engagement balloon 22 are provided at the distal end section 10a of the insertion section 10 in this order from the front in the advance direction of the distal end section 10a.

Further, there is also provided a holding balloon 23 which holds the position of the distal end section 10a of the insertion section 10 substantially at the center inside the tube at the time when the drive balloon 20 and the engagement balloon 22 are not brought into contact with the tube wall.

The drive balloon 20, the engagement balloon 22, and the holding balloon 23 are all made of freely expandable and contractible latex rubber.

The engagement balloon 22 is a balloon having an expansion characteristic of being able to come into contact with the inner wall surface of the tube wall at the time of expansion so as to engage with the inner wall surface, while the drive balloon 20 is a balloon having an expansion characteristic of being unable to come into contact with the inner wall surface of the tube wall even at the time of expansion as long as the distal end section 10a is located substantially at the central position of the cross section of the tube.

Further, it is preferred that the shapes of the drive balloon 20 and the engagement balloon 22 are different from each other.

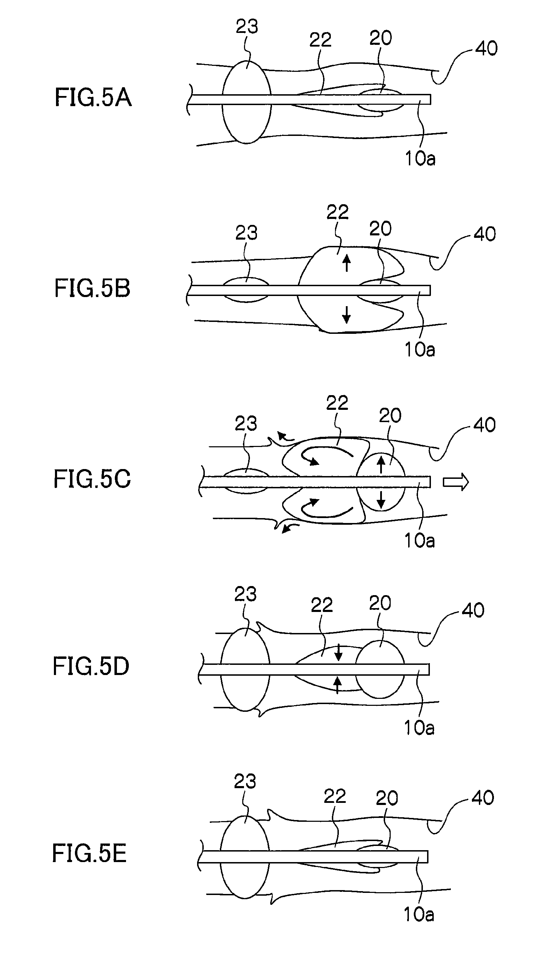

Note that the engagement balloon 22 at the time of contraction may not necessarily cover the drive balloon 20 as shown in Fig. 2. As will be described below, the engagement balloon 22 may cover the drive balloon 20 at least at the time when the engagement balloon 22 is expanded to engage with an intestinal wall 40 (see Figs. 5A to 5E).

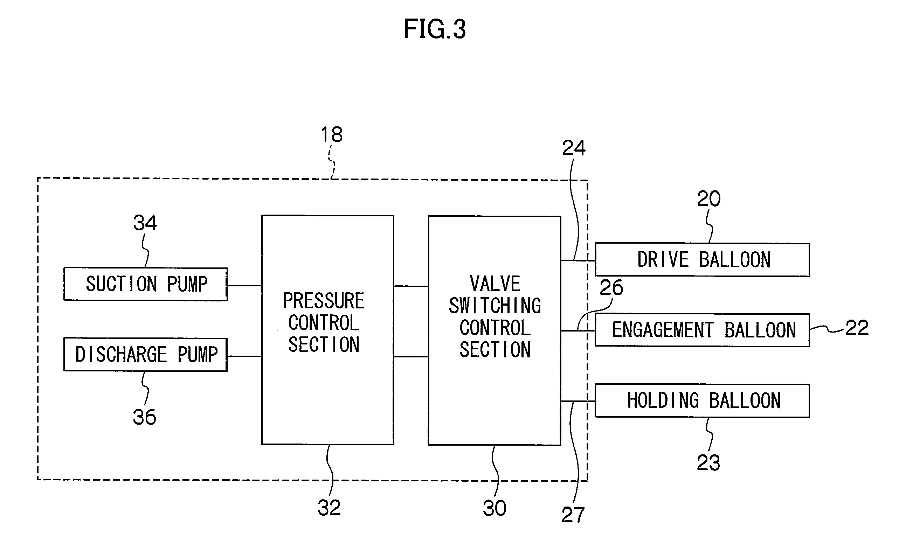

Further, Fig. 3 is a block diagram of the balloon control apparatus 18 which controls the pressure of the drive balloon 20 and the engagement balloon 22. As shown in Fig. 3, the drive balloon 20, the engagement balloon 22, and the holding balloon 23 are configured so as to enable the internal pressure thereof to be independently adjusted, and are connected to a suction pump 34 and a discharge pump 36 via a valve switching control section 30 and a pressure control section 32.

As shown in Fig. 2, a gas supply tube 24 which communicates with the drive balloon 20 to supply gas to the drive balloon 20, a gas supply tube 26 which communicates with the engagement balloon 22 to supply gas to the engagement balloon 22, and a gas supply tube 27 which communicates with the holding balloon 23 to supply gas to the holding balloon 23, are provided in the inside of the distal end section 10a. The gas supply tube 24, the gas supply tube 26, and the gas supply tube 27 are connected to the balloon control apparatus 18 (see Fig. 1 and Fig. 3) through the inside of the bending section 10b and the soft section 10c, and the code 14 (see Fig. 1).

Note that in the flow of the propulsion operation as will be described below, the propulsion operation is performed in such a manner that the opening and closing of valves (not shown) connected to the respective balloons are controlled by the valve switching control section 30, and that the suction pump 34 and the discharge pump 36 are controlled by the pressure control section 32.

### <Flow of propulsion operation>

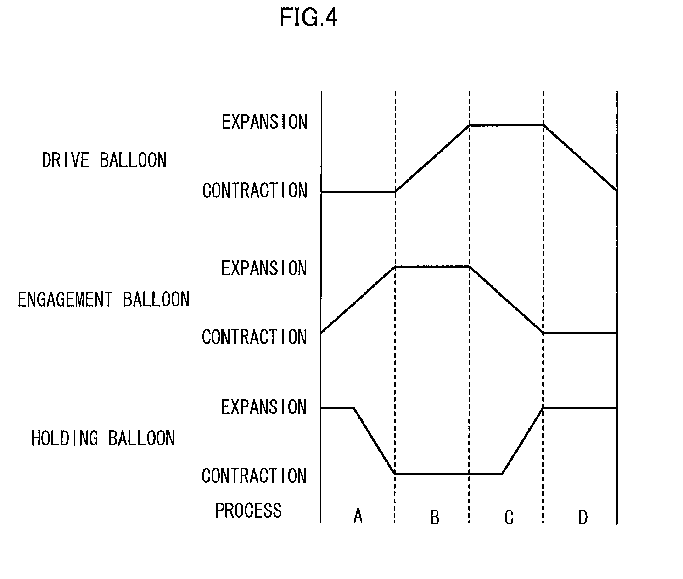

Fig. 4 is a time chart of a propulsion operation in example 1 according to the first embodiment. Note that, as will be described below, a propulsion operation in example 2 according to the first embodiment is also represented by the time chart in Fig. 4.

Further, Figs. 5A to 5E are schematic sectional views showing states of expansion and contraction of the respective balloons in example 1 according to the first embodiment in correspondence with the time chart of the propulsion operation shown in Fig. 4.

Note that the present example will be described by assuming that the propulsion operation is the forward movement operation.

First, there is considered a case where the distal end section 10a of the electronic endoscope 1 is inserted into a measuring object (here, for example, the large intestine) in the state in which both the drive balloon 20 and the engagement balloon 22 are contracted. Note that at this time, the holding balloon 23 is expanded so as to engage with the intestinal wall 40.

Then, in the state in which the drive balloon 20 and the engagement balloon 22 are both contracted as shown in Fig. 5A, the engagement balloon 22 is filled with the gas and expanded so as to engage with the intestinal wall 40, while the holding balloon 23 is contracted (process A of Fig. 4). The states of expansion and contraction of the balloons at this time can be represented as shown in Fig. 5B. As shown in Fig. 5B, the engagement balloon 22 is expanded so as to cover the drive balloon 20, and is in the state of engaging with the intestinal wall 40. Further, it is considered here that, when the engagement balloon 22 is expanded to come into contact with the intestinal wall 40, a portion of the engagement balloon 22, which portion fills between the insertion section 10 and the intestinal wall 40, is a first portion, and that a portion of the engagement balloon 22, which portion is in contact with the intestinal wall 40, is a second portion.

Next, the drive balloon 20 is filled with the gas so as to be expanded (process B in Fig. 4). The states of expansion and contraction of the balloons at this time can be represented as shown in Fig. 5C.

As shown in Fig. 5C, as the drive balloon 20 is expanded in the state where the engagement balloon 22 is held to engage with the intestinal wall 40, the drive balloon 20 gradually presses the engagement balloon 22. Then, the engagement balloon 22 is pushed so that the surface of the engagement balloon 22 is gradually successively fed or moved backward in the advance direction of the distal end section 10a. Further, when it is considered that the engagement balloon 22 includes the first portion and the second portion as described above, it can be considered that a part of the first portion, which part is located on the front side in the advance direction of the distal end section 10a and on the side of the intestinal wall 40, is brought into contact with the intestinal wall 40 so as to be pressed to become the second portion. Thereby, the engagement balloon 22 applies to the intestinal wall 40 backward pressing force (shown by the black arrow in Fig. 5C) in the advance direction of the distal end section 10a.

That is, the engagement balloon 22 is fed backward in the advance direction of the distal end section 10a while being in contact with the intestinal wall 40, similarly to an endless track like a so-called caterpillar (registered trademark).

Thereby, the intestinal wall 40 is drawn backward in the advance direction of the distal end section 10a. Therefore, as represented by the white arrow in Fig. 5C, the distal end section 10a of the electronic endoscope 1 is propelled (moved) forward in the advance direction relatively to the intestinal wall 40.

Next, the engagement balloon 22 is contracted by sucking the gas therefrom, so as to be separated from the intestinal wall 40, while the holding balloon 23 is expanded to engage with the intestinal wall 40 (process C of Fig. 4). The states of expansion and contraction of the balloons at this time can be represented as shown in Fig. 5D.

Note that in the present embodiment, it is considered that even if the engagement balloon 22 is separated from the intestinal wall 40 at this time, the state where the intestinal wall 40 is drawn backward in the advance direction of the distal end section 10a is maintained.

Further, in process C in Fig. 4, it can be considered that in dependence upon the bending amount of the intestinal wall 40, the engagement balloon 22 is not separated from the intestinal wall 40 and remains to be in contact with the intestinal wall 40. However, even in such case, it is only necessary that the engagement balloon 22 does not apply the engagement force to the intestinal wall 40.

Next, the drive balloon 20 is contracted by sucking the gas therefrom (process D of Fig. 4). Thereby, as shown in Fig. 5E, the states of expansion and contraction of the balloons are returned to the above described states shown in Fig. 5A.

Thereafter, when the forward movement operation is continued, the processes from process A to process D are repeated.

Note that when the arrangement order of the drive balloon 20 and the engagement balloon 22 is reversed and when the engagement balloon 22 and the drive balloon 20 are arranged in this order from the front in the advance direction of the distal end section 10a of the insertion section 10, the backward movement operation can be performed.

Further, instead of using balloons like the drive balloon 20 and the engagement balloon 22, an expansion/contraction member expandable and contractible into a desired shape and size made of a material such as polyurethane having relatively low expansion and contraction properties, and cloth may be used. In the case of use of a material having low expansion and contraction properties, a desired diameter (shape) required for propulsion is preferably made as an initial shape, since change in the expansion amount with respect to the pressure is small.

The above is the description of the specific propulsion operation in example 1 according to the first embodiment.

Note that the combination of the drive balloon 20 and the engagement balloon 22 may also be provided at a plurality of places.

### (Example 2)

In example 2, unlike example 1, a balloon having directivity in the direction of deformation at the time of expansion and contraction is used in place of the drive balloon 20.

### <Configuration of actuator for moving body in tube>

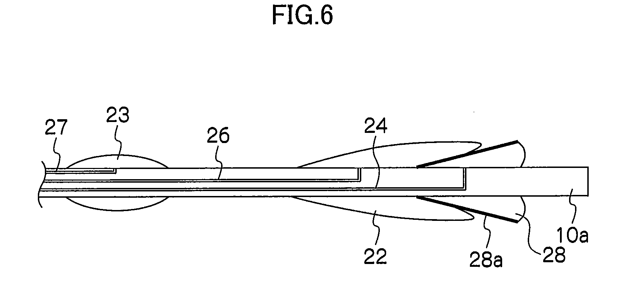

Fig. 6 is an enlarged sectional view showing the distal end section 10a of the insertion section 10 in example 2 according to the first embodiment. As shown in Fig. 6, example 2 is different from example 1 in that a directed drive balloon 28 is provided in place of the drive balloon 20.

The directed drive balloon 28 and the engagement balloon 22 are formed in the entire circumferential direction of the insertion section 10. It is preferred that the directed drive balloon 28 is arranged adjacent to the inside of the engagement balloon 22.

The directed drive balloon 28 is made of natural rubber such as latex rubber.
However, the directed drive balloon 28 is configured to have a portion having a thickness larger than the other portion thereof, and thereby, has directivity in the direction of deformation at the time of expansion and contraction. In the present example, the portion of the surface 28a which is brought into contact with the engagement balloon 22 is formed to have a larger thickness than the other portion as shown in Fig. 6. Note that the directivity in the direction of deformation at the time of expansion and contraction may also be imparted to the directed drive balloon 28 by providing a low expansion material in the portion of the surface 28a which is brought into contact with the engagement balloon 22. On the other hand, the entire part of the engagement balloon 22 is made of natural rubber such as freely expandable and contractible latex rubber.

Note that the directed drive balloon 28 and the engagement balloon 22 may have an axially symmetrical shape which is uniform in the circumferential direction of the insertion section 10, and may also have an axially asymmetrical shape which is not uniform in the circumferential direction of the insertion section 10.

Further, the block configuration and operation of the balloon control apparatus 18 which controls the pressure of the respective balloons in example 2 are in common with those of example 1 shown in Fig. 3 except that the balloon configuration is changed to the configuration by the directed drive balloon 28 and the engagement balloon 22.

### <Flow of propulsion operation>

The time chart of specific propulsion operation in example 2 according to the first embodiment is in common with the time chart in Fig. 4 of example 1.

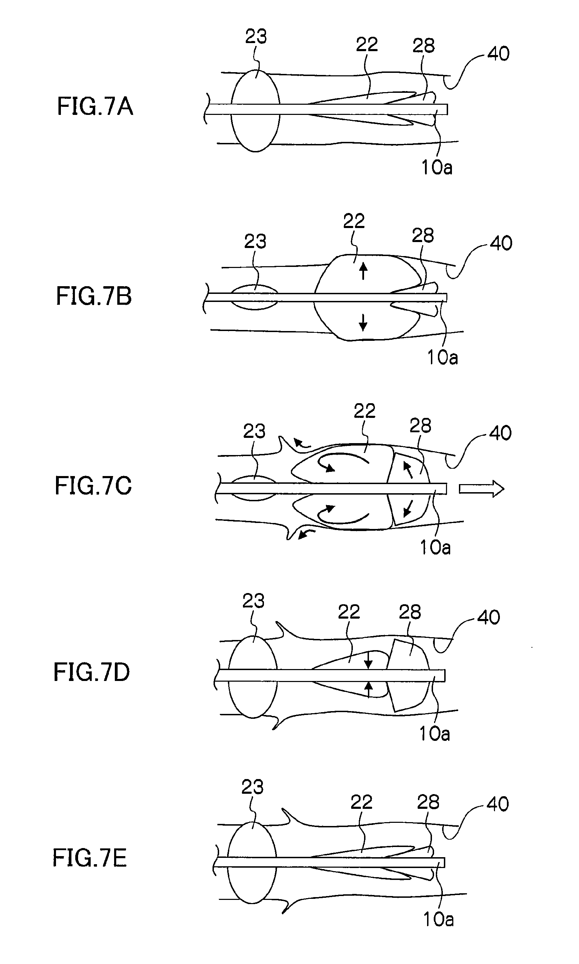

Figs. 7A to 7E are schematic sectional views showing states of expansion and contraction of the respective balloons in example 2 according to the first embodiment in correspondence with the time chart of the propulsion operation shown in Fig. 4.

The propulsion operation of example 2 according to the first embodiment is substantially the same as that of example 1 when the drive balloon 20 is replaced by the directed drive balloon 28. However, the directed drive balloon 28 is different from the drive balloon 20 of example 1 in that the thickness of the portion of the surface 28a, which is brought into contact with the engagement balloon 22, is set larger than the other portion.

Thereby, as shown in Fig. 7C, the directed drive balloon 28 is expanded in the direction of the engagement balloon 22 at the time of expansion, and hence can easily press the engagement balloon 22 at the time of expansion as compared with the drive balloon 20 of example 1.

This increases the amount by which the surface of the engagement balloon 22 is pressed so as to be successively fed backward in the advance direction of the distal end section 10a. Alternatively, when it is considered that the engagement balloon 22 includes the first portion and the second portion as described above, it can be considered that the amount of the part of the first portion, which part is located on the front side in the advance direction of the distal end section 10a and on the side of the intestinal wall 40, and which part is brought into contact with the intestinal wall 40 so as to be pressed to become the second portion, is increased. This increases the amount by which the intestinal wall 40 is drawn backward in the advance direction of the distal end section 10a.

With the above operation, as shown by the white arrow in Fig. 7C, the amount, by which the distal end section 10a of the electronic endoscope 1 is propelled (moved) forward in the advance direction relatively to the intestinal wall 40, is increased as compared with example 1.

Note that the reverse movement operation can be performed when the arrangement order of the directed drive balloon 28 and the engagement balloon 22 is reversed and when the engagement balloon 22 and the directed drive balloon 28 are arranged in this order from the front in the advance direction of the distal end section 10a of the insertion section 10.

Further, instead of using balloons like the directed drive balloon 28 and the engagement balloon 22, it may also be possible to use expansion/contraction members, which are made of a material like polyurethane having relatively low expansion and contraction properties and cloth, and which can be expanded and contracted into a desired shape and size. In the case of using a material having low expansion and contraction properties, change in the expansion amount with respect to the pressure is small, and therefore, a desired diameter (shape) required for propulsion is preferably made as an initial shape.

Note that the combination of the directed drive balloon 28 and the engagement balloon 22 may also be provided at a plurality of places.

As described above, in the present embodiment, the control is performed such that after the engagement balloon 22 is expanded to engage with the intestinal wall 40, the drive balloon 20 or the directed drive balloon 28 is expanded to press the engagement balloon 22. Thereby, the distal end section 10a can be moved by surely drawing the intestinal wall 40, while the engagement balloon 22 is prevented from sliding on the intestinal wall 40.

Further, in the present embodiment, in step B of Fig. 4, internal pressure P2 at the time of expanding the drive balloon 20 or the directed drive balloon 28 is preferably controlled to be equal to or greater than the internal pressure P1 of the engagement balloon 22 in the state of engaging with the intestinal wall 40.

For example, in the case of example 1, when the engagement balloon 22 in the state of engaging with the intestinal wall 40 is expanding so as to cover the drive balloon 20 as shown in Fig. 5B, if the internal pressure P2 of the drive balloon 20 is lower than the internal pressure P1 of the engagement balloon 22, the drive balloon 20 is pressed by the engagement balloon 22 and cannot expand to a sufficient size, and an expansion diameter of the drive balloon 20 becomes small, which becomes the factor of causing reduction in propulsive force (drive force).

In contrast with this, by performing control so that the internal pressure P2 of the drive balloon 20 becomes equal to or greater than the internal pressure P1 of the engagement balloon 22, the drive balloon 20 can be expanded to a sufficient size (expansion diameter) without being pressed by the engagement balloon 22, and reduction in propulsive force can be prevented.

The propulsive force of the method (turning balloon method) of performing control, so as to press the engagement balloon 22 in the state of engaging with the intestinal wall 40 by expanding the drive balloon 20 or the directed drive balloon 28 as in the present embodiment, tends to increase proportionally to the expansion amount (expansion diameter) of the drive balloon 20 or the directed drive balloon 28.

Generally, the internal pressure P1 of the engagement balloon 22 in the state of engaging with the intestinal wall 40 is controlled to be about 5.6 to 8.2 [kPa].
Therefore, the internal pressure P2 of the drive balloon 20 or the directed drive balloon 28 is preferably controlled to be at least 5.6 [kPa], and more preferably 8.2 [kPa] or more.

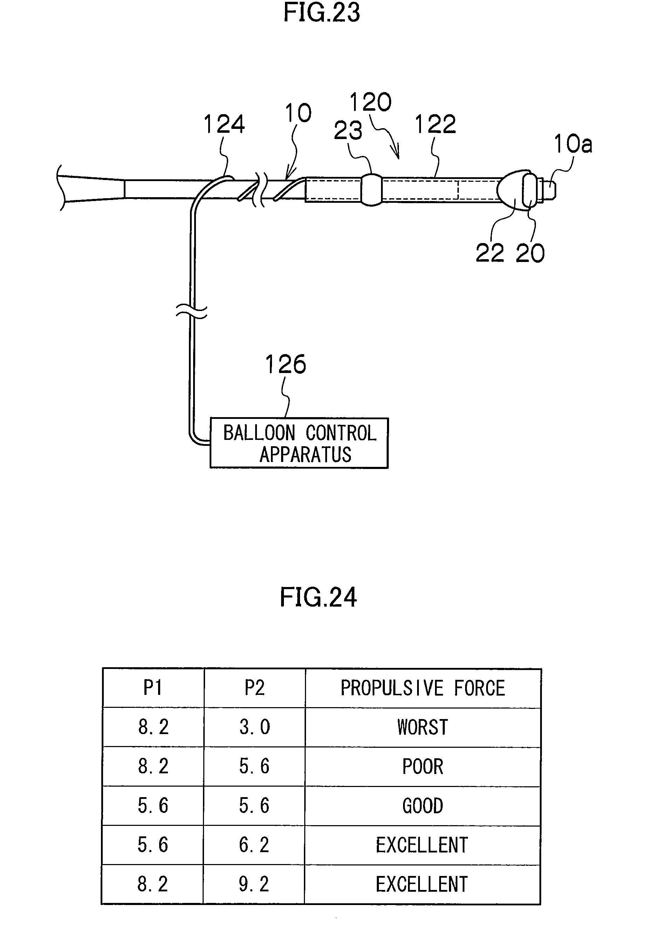

Here, as one example, the relationship of the internal pressure P1 of the engagement balloon 22 and the internal pressure P2 of the drive balloon 20 and the propulsive force is shown in Fig. 24. As is understood from Fig. 24, the propulsive force obtained in the case of satisfying the relationship of P1>P2 is insufficient (evaluation "Poor" or "Worst"), but in the case of satisfying the relationship of P1≤P2, sufficient propulsive force can be obtained (evaluation "Excellent" or "Good").

The internal pressure P1 and the internal pressure P2 of the respective balloons are controlled by the balloon control apparatus 18 (see Figs. 1 and 3). In the present embodiment, a pressure detecting device (not illustrated) which detects the internal pressure of each of the balloons is provided, and the balloon control apparatus 18 performs control so that the internal pressure P1 and the internal pressure P2 of the respective balloons are in the predetermined pressure ranges respectively based on the detection result by the pressure detecting device. Thereby, the pressure control with high precision can be performed, and the propulsive force can be efficiently obtained.

Further, engagement balloons that have been practically used can be applied to the engagement balloon 22, which proves high technical feasibility. Further, the engaging force (grip force to the intestinal wall 40) of the engagement balloon 22 that has been already practically used can be applied as it is.

Further, the engagement balloon 22, the drive balloon 20, and the directed drive balloon 28 are separately provided, which makes it possible to carry out the technical development specifically directed to the respective functions. For example, the drive balloon 20 and the directed drive balloon 28 are not brought into contact with the intestinal wall 40, and hence importance can be placed on the directivity in the material, the shape, the size, and the like, of the drive balloon 20 and the directed drive balloon 28.

### [Second embodiment]

Next, a second embodiment will be described. In the second embodiment, the number of balloons is set to three.

### <Configuration actuator for moving body in tube>

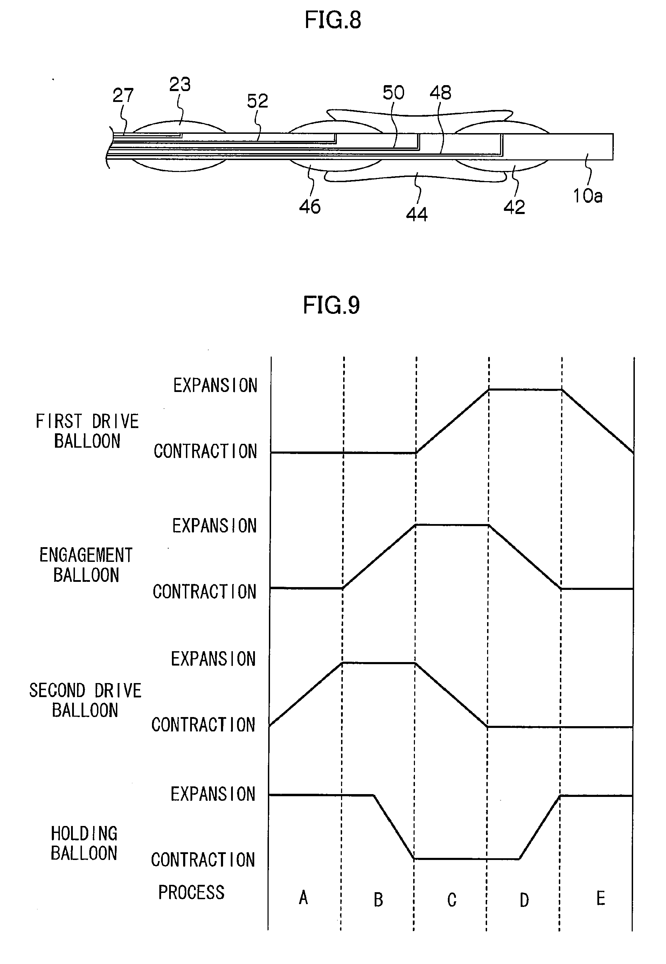

Fig. 8 is an enlarged sectional view showing the distal end section 10a of the insertion section 10 according to a second embodiment. As shown in Fig. 8, three balloons of a first drive balloon 42 and an engagement balloon 44, and a second drive balloon 46 are arranged side by side at the distal end section 10a of the insertion section 10. The second drive balloon 46 is arranged on the side opposite to the first drive balloon 42 across the engagement balloon 44. Further, the first drive balloon 42, the engagement balloon 44, and the second drive balloon 46 are fixed to the insertion section 10.

The entire part of each of the first drive balloon 42, the engagement balloon 44, and the second drive balloon 46 is made of freely expandable and contractible latex rubber.

Note that the first drive balloon 42, the engagement balloon 44, and the second drive balloon 46 are formed in the entire circumferential direction of the insertion section 10. Further, the first drive balloon 42, the engagement balloon 44, and the second drive balloon 46 may have an axially symmetrical shape which is uniformly formed in the circumferential direction of the insertion section 10, or may have an axially asymmetrical shape which is not uniformly formed in the circumferential direction of the insertion section 10.

Further, it is preferred that there are at least a difference between the shapes of the engagement balloon 44 and the first drive balloon 42, and a difference between the shapes of the engagement balloon 44 and the second drive balloon 46.

Note that the engagement balloon 44 at the time of contraction needs not necessarily cover the first drive balloon 42 and the second drive balloon 46 as shown in Fig. 8, and that as will be described below, the engagement balloon 44 may cover the first drive balloon 42 and the second drive balloon 46 at least at the time when the engagement balloon 44 is expanded to engage with the intestinal wall 40.

Note that the block configuration and operation of the balloon control apparatus 18 which controls the pressure of the respective balloons in the second embodiment are in common with those of example 1 according to the first embodiment shown in Fig. 3 except that the balloon configuration is changed to the configuration by the first drive balloon 42, the engagement balloon 44, and the second drive balloon 46.

As shown in Fig. 8, a gas supply tube 48 which communicates with the first drive balloon 42 to supply gas to the first drive balloon 42, a gas supply tube 50 which communicates with the engagement balloon 44 to supply gas to the engagement balloon 44, and a gas supply tube 52 which communicates with the second drive balloon 46 to supply gas to the second drive balloon 46 are provided in the inside of the distal end section 10a. The gas supply tube 48, the gas supply tube 50, and the gas supply tube 52 are connected to the balloon control apparatus 18 (see Fig. 1 and Fig. 3) through the inside of the bending section 10b and the soft section 10c, and the code 14 (see Fig. 1).

### <Flow of propulsion operation>

First, a forward movement operation will be described.

The second embodiment includes total three balloons of the first drive balloon 42, the engagement balloon 44, and the second drive balloon 46.

First, the same forward movement operation as the forward movement operation performed by using the drive balloon 20 and the engagement balloon 22 in example 1 according to the first embodiment can be performed by using the first drive balloon 42 and the engagement balloon 44 among the three balloons. Note that the detailed description of the operation flow is duplicated and hence is omitted.

Next, there will be described an operation at the time when the forward movement operation is performed by using a total of three balloons of the first drive balloon 42, the engagement balloon 44, and the second drive balloon 46.

Fig. 9 is a time chart at the time when the forward movement operation is performed by using the three balloons in the second embodiment.

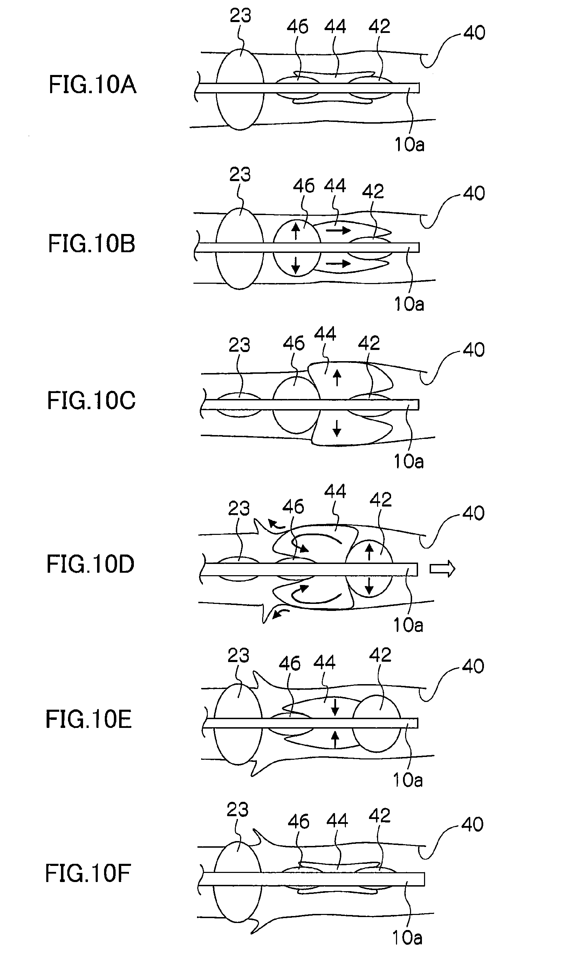

Further, Figs. 10A to 10F are schematic sectional views showing states of expansion and contraction of the respective balloons in correspondence with the time chart shown in Fig. 9.

First, there is considered a case where the distal end section 10a of the electronic endoscope 1 is inserted into a measuring object (here, for example, the large intestine) in the state where the first drive balloon 42, the engagement balloon 44, and the second drive balloon 46 are all contracted. Note that the holding balloon 23 is expanded to engage with the intestinal wall 40 at this time.

Then, in the state where the first drive balloon 42, the engagement balloon 44, and the second drive balloon 46 are all contracted as shown in Fig. 10A, the second drive balloon 46 is expanded by being filled with the gas (process A in Fig. 9). The states of expansion and contraction of the respective balloons can be represented as shown in Fig. 10B. When the second drive balloon 46 is expanded as shown in Fig. 10B, the engagement balloon 44 is pushed to the side of the first drive balloon 42, so as to cover the first drive balloon 42.

Next, when the engagement balloon 44 is expanded by being filled with the gas while the holding balloon 23 is contracted, the engagement balloon 44 is made to engage with the intestinal wall 40 (process B in Fig. 9). The states of expansion and contraction of the respective balloons at this time can be represented as shown in Fig. 10C.

Further, it is considered here that when the engagement balloon 44 is expanded to come into contact with the intestinal wall 40, a portion of the engagement balloon 44, which portion fills between the insertion section 10 and the intestinal wall 40, is set as a first portion, and that a portion of the engagement balloon 44, which portion is in contact with the intestinal wall 40, is set as a second portion.

Next, the first drive balloon 42 is expanded by being filled with the gas, and at the same time, the second drive balloon 46 is contracted by sucking the gas therefrom (process C in Fig. 9). The states of expansion and contraction of the respective balloons at this time can be represented as shown in Fig. 10D.

As shown in Fig. 10D, as the first drive balloon 42 is expanded, the first drive balloon 42 gradually presses the engagement balloon 44. Further, the second drive balloon 46 is gradually contracted, so that the surface of the engagement balloon 44 is pushed so as to be successively fed (or moved) backward in the advance direction of the distal end section 10a in the state where the surface of the engagement balloon 44 is in contact with the intestinal wall 40. Further, when it is considered that the engagement balloon 44 includes the first portion and the second portion as described above, it can be considered that a part of the first portion, which is located on the front side of the advance direction of the distal end section 10a and on the side of the intestinal wall 40, is brought into contact with the intestinal wall 40 so as to be pushed to become the second portion. Thereby, the engagement balloon 44 gives to the intestinal wall 40 the backward pressing force (represented by the black arrow in Fig. 10D) in the advance direction of the distal end section 10a.

That is, the engagement balloon 44 is fed backward in the advance direction of the distal end section 10a while being in contact with the intestinal wall 40, similarly to an endless track like a so-called caterpillar (registered trademark).

Thereby, the intestinal wall 40 is drawn backward in the advance direction of the distal end section 10a. Therefore, as represented by the white arrow in Fig. 10D, the distal end section 10a of the electronic endoscope 1 is propelled (moved) forward in the advance direction relatively to the intestinal wall 40.

Here, as described above, before process B in Fig. 9, in which the engagement balloon 44 is made to engage with the intestinal wall 40, the engagement balloon 44 is pushed, in process A in Fig. 9, to the side of the first drive balloon 42 due to the expansion of the second drive balloon 46 so as to be in the state of covering the first drive balloon 42. For this reason, in process C in Fig. 9, the amount, by which the engagement balloon 44 is successively fed backward in the advance direction of the distal end section 10a in the state where the surface of the engagement balloon 44 is in contact with the intestinal wall 40, is increased as compared with the case where the two balloons described above are used (the case where the two balloons are used in the first embodiment or the second embodiment).

Therefore, as represented by the white arrow in Fig. 10D, the amount by which the intestinal wall 40 is drawn backward in the advance direction of the distal end section 10a is increased, so that the amount by which the distal end section 10a is propelled (moved) forward in the advance direction relatively to the intestinal wall 40 is increased as compared with the case where the two balloons described above are used.

Next, the engagement balloon 44 is contracted by sucking the gas therefrom, so as to be separated from the intestinal wall 40, while the holding balloon 23 is expanded so as to engage with the intestinal wall 40 (process D in Fig. 9). The states of expansion and contraction of the balloons at this time are represented as shown in Fig. 10E.

Next, the first drive balloon 42 is contracted by sucking the gas therefrom (process E in Fig. 9). Thereby, as shown in Fig. 10F, the states of expansion and contraction of the balloons are returned to the above described states shown in Fig. 10A.

Thereafter, when the forward movement operation is continued, the processes from process A to process E are repeated.

The above is the description of the propulsion operation by using the three balloons in the second embodiment.

Next, a backward movement operation will be described.

The actuator for the moving body in tube can be moved backward by using two balloons of the engagement balloon 44 and the second drive balloon 46 among the three balloons. Thus, the detailed flow of the operation will be described below.

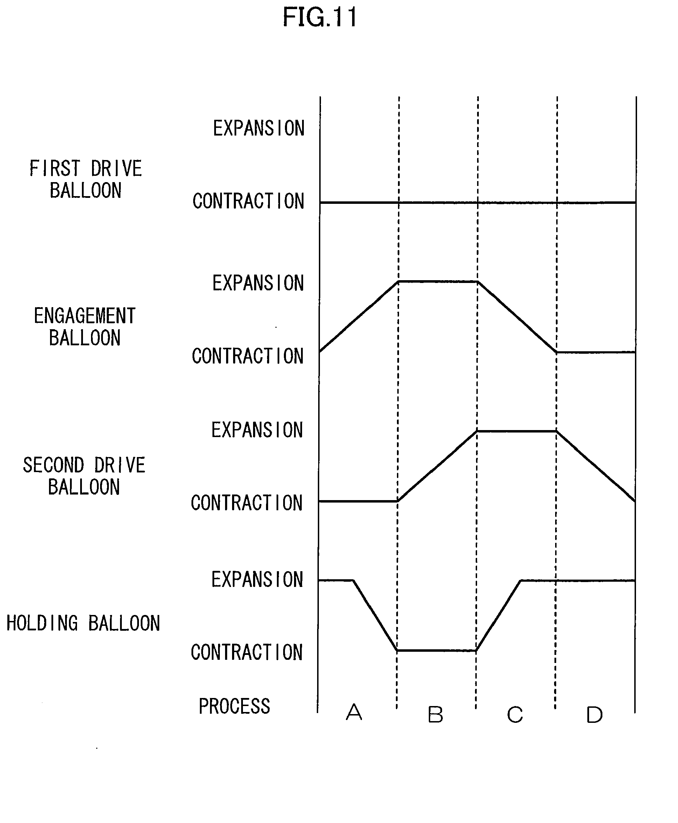

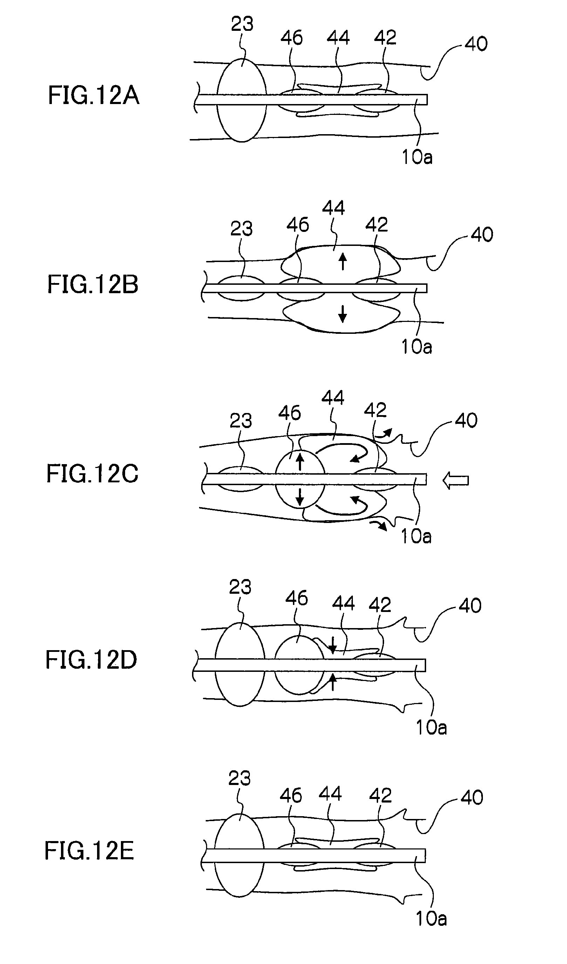

Fig. 11 is a time chart at the time when the backward movement operation is performed by using two balloons in the second embodiment. Further, Figs. 12A to 12E are schematic sectional views showing states of expansion and contraction of the respective balloons in correspondence with the time chart of the forward movement operation shown in Fig. 11.

First, there is considered a case where the distal end section 10a of the electronic endoscope 1 is inserted into a measuring object (here, for example, the large intestine) in the state in which both the engagement balloon 44 and the second drive balloon 46 are contracted. Note that at this time, the holding balloon 23 is expanded so as to engage with the intestinal wall 40.

Then, from the state where the first drive balloon 42, the engagement balloon 44, and the second drive balloon 46 are all contracted as shown in Fig. 12A, the engagement balloon 44 is filled with the gas and expanded so as to engage with the intestinal wall 40, while the holding balloon 23 is contracted (process A of Fig. 11). The states of expansion and contraction of the balloons at this time can be represented as shown in Fig. 12B. Further, it is considered here that a portion of the engagement balloon 44, which portion fills between the insertion section 10 and the intestinal wall 40 at the time when the engagement balloon 44 is expanded to come into contact with the intestinal wall 40, is set as a first portion, and that a portion of the engagement balloon 44, which portion is in contact with the intestinal wall 40, is set as a second portion.

Next, the second drive balloon 46 is filled with the gas so as to be expanded (process B in Fig. 11). The states of expansion and contraction of the balloons at this time can be represented as shown in Fig. 12C.

As shown in Fig. 12C, as the second drive balloon 46 is expanded, the second drive balloon 46 gradually presses the engagement balloon 44. Then, the engagement balloon 44 is pushed so that the surface of the engagement balloon 44 is successively fed or moved forward in the advance direction of the distal end section 10a. Further, when it is considered that the engagement balloon 44 includes the first portion and the second portion as described above, it can be considered that a part of the first portion, which part is located on the back side in the advance direction of the distal end section 10a and on the side of the intestinal wall 40, is brought into contact with the intestinal wall 40 so as to be pressed to become the second portion. Thereby, the engagement balloon 44 applies to the intestinal wall 40 forward pressing force (shown by the black arrow in Fig. 12C) in the advance direction of the distal end section 10a.

That is, the engagement balloon 44 is fed forward in the advance direction of the distal end section 10a while being in contact with the intestinal wall 40, similarly to an endless track like a so-called caterpillar (registered trademark).

Thereby, the intestinal wall 40 is drawn forward in the advance direction of the distal end section 10a. Therefore, as represented by the white arrow in Fig. 12C, the distal end section 10a of the electronic endoscope 1 is propelled (moved) backward in the advance direction relatively to the intestinal wall 40.

Next, the engagement balloon 44 is contracted by sucking the gas therefrom, so as to be separated from the intestinal wall 40, while the holding balloon 23 is expanded to engage with the intestinal wall 40 (process C of Fig. 11). The states of expansion and contraction of the balloons at this time are represented as shown in Fig. 12D.

Next, the second drive balloon 46 is contracted by sucking the gas therefrom (process D of Fig. 11). Thereby, as shown in Fig. 12E, the states of expansion and contraction of the balloons are returned to the above described states shown in Fig. 12A.

Thereafter, when the backward movement operation is continued, the processes from process A to process D are repeated.

The above is the description of the backward movement operation performed by using the two balloons in the second embodiment.

Next, there will be described a backward movement operation performed by using a total three balloons including the first drive balloon 42 in addition to the second drive balloon 46 and the engagement balloon 44.

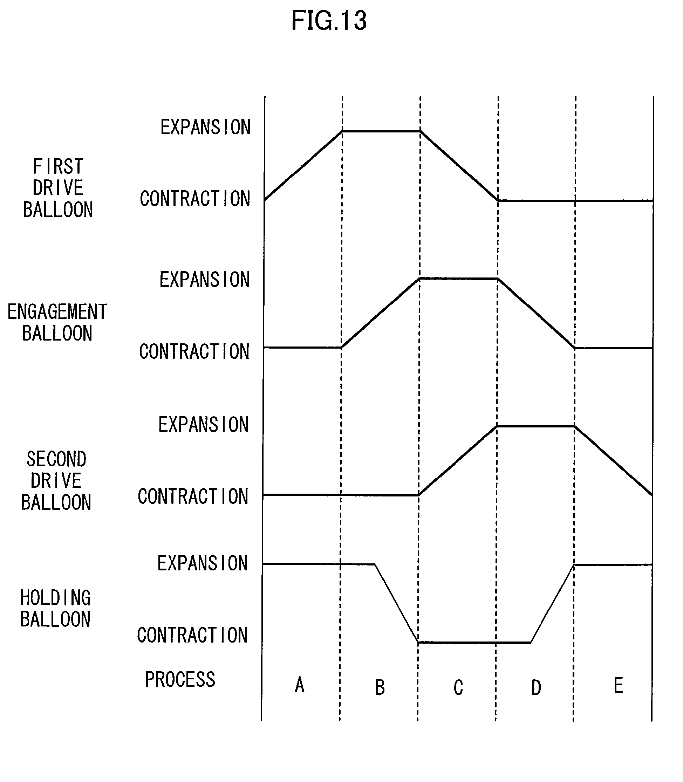

Fig. 13 is a time chart at the time when the backward movement operation is performed by using the three balloons in the second embodiment.

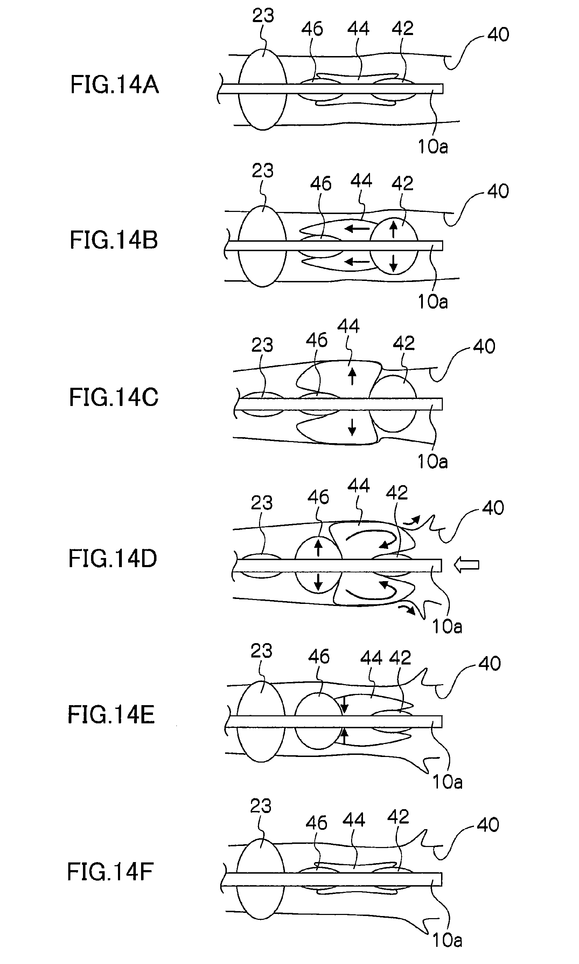

Further, Figs. 14A to 14F are schematic sectional views showing states of expansion and contraction of the respective balloons in correspondence with the time chart shown in Fig. 13.

First, there is considered a case where the distal end section 10a of the electronic endoscope 1 is inserted into a measuring object (here, for example, the large intestine) in the state where the first drive balloon 42, the engagement balloon 44, and the second drive balloon 46 are all contracted. Note that the holding balloon 23 is expanded to engage with the intestinal wall 40 at this time.

Then, in the state where the first drive balloon 42, the engagement balloon 44, and the second drive balloon 46 are all contracted as shown in Fig. 14A, the first drive balloon 42 is expanded by being filled with the gas (process A in Fig. 13). The states of expansion and contraction of the respective balloons at this time can be represented as shown in Fig. 14B. When the first drive balloon 42 is expanded as shown in Fig. 14B, the engagement balloon 44 is pushed to the side of the second drive balloon 46, so as to cover the second drive balloon 46.

Next, when the engagement balloon 44 is expanded by being filled with the gas while the holding balloon 23 is contracted, the engagement balloon 44 is made to engage with the intestinal wall 40 (process B in Fig. 13). The states of expansion and contraction of the respective balloons at this time can be represented as shown in Fig. 14C. Further, it is considered here that when the engagement balloon 44 is brought into contact with the intestinal wall 40, a portion of the engagement balloon 44, which portion fills between the insertion section 10 and the intestinal wall 40, is set as a first portion, and that a portion of the engagement balloon 44, which portion is in contact with the intestinal wall 40, is set as a second portion.

Next, the second drive balloon 46 is expanded by being filled with the gas, and at the same time, the first drive balloon 42 is contracted by sucking the gas therefrom (process C in Fig. 13). The states of expansion and contraction of the respective balloons at this time can be represented as shown in Fig. 14D.

As shown in Fig. 14D, as the second drive balloon 46 is expanded, and as the first drive balloon 42 is contracted, the second drive balloon 46 gradually presses the engagement balloon 44. Then, the surface of the engagement balloon 44 is pushed so as to be successively fed or moved forward in the advance direction of the distal end section 10a. Further, when it is considered that the engagement balloon 44 includes the first portion and the second portion as described above, it can be considered that a part of the first portion, which part is located on the back side in the advance direction of the distal end section 10a and on the side of the intestinal wall 40, is brought into contact with the intestinal wall 40 and pushed so as to become the second portion. Thereby, the engagement balloon 44 applies to the intestinal wall 40 the forward pressing force (represented by the black arrow in Fig. 14D) in the advance direction of the distal end section 10a.

That is, the engagement balloon 44 is fed forward in the advance direction of the distal end section 10a while being in contact with the intestinal wall 40, similarly to an endless track like a so-called caterpillar (registered trademark).

Thereby, the intestinal wall 40 is drawn forward in the advance direction of the distal end section 10a. Therefore, as represented by the white arrow in Fig. 14D, the distal end section 10a of the electronic endoscope 1 is propelled (moved) backward in the advance direction relatively to the intestinal wall 40.

Here, as described above, before process B in Fig. 13, in which the engagement balloon 44 is made to engage with the intestinal wall 40, the engagement balloon 44 is pushed, in process A in Fig. 13, to the side of the second drive balloon 46 due to the expansion of the first drive balloon 42 so as to be in the state of covering the second drive balloon 46. For this reason, in process C in Fig. 13, the amount, by which the engagement balloon 44 is successively fed forward in the advance direction of the distal end section 10a in the state where the surface of the engagement balloon 44 is in contact with the intestinal wall 40, is increased as compared with the case where the two balloons described above are used (the case where the two balloons are used in the first embodiment or the second embodiment).

Therefore, the amount by which the intestinal wall 40 is drawn forward in the advance direction of the distal end section 10a is increased, so that the amount by which the distal end section 10a is propelled (moved) backward in the advance direction relatively to the intestinal wall 40 as shown by the white arrow in Fig. 14D is increased as compared with the case where the two balloons described above are used.

Next, the engagement balloon 44 is contracted by sucking the gas therefrom, so as to be separated from the intestinal wall 40, while the holding balloon 23 is expanded so as to engage with the intestinal wall 40 (process D in Fig. 13). The states of expansion and contraction of the balloons at this time are represented as shown in Fig. 14E.

Next, the second drive balloon 46 is contracted by sucking the gas therefrom (process E in Fig. 13). Thereby, as shown in Fig. 14F, the states of expansion and contraction of the balloons are returned to the above described states shown in Fig. 14A.

Thereafter, when the backward movement operation is continued, the processes from process A to process E are repeated.

The above is the description of the backward movement operation by using the three balloons in the second embodiment.

Note that in the second embodiment, the directed drive balloon 28 described in example 2 according to the first embodiment may also be used as the first drive balloon 42 and the second drive balloon 46 in place of the respective drive balloons.

Further, instead of using balloons like the first drive balloon 42, the engagement balloon 44, and the second drive balloon 46, it may also be possible to use expansion/contraction members which are made of a material like polyurethane having relatively low expansion and contraction properties and cloth and which can be expanded and contracted into a desired shape and size. In the case of using a material with low expansion and contraction properties, a desired diameter (shape) required for propulsion is preferably made as an initial shape, since the change in the expansion amount with respect to the pressure is small.

Note that the combination of the first drive balloon 42, the engagement balloon 44, and the second drive balloon 46 may also be provided at a plurality of places.

As described above, in the present embodiment, the control is performed such that before the engagement balloon 44 is expanded to engage with the intestinal wall 40, the second drive balloon 46 is expanded to press the engagement balloon 44, and that after the engagement balloon 44 is expanded to engage with the intestinal wall 40, the first drive balloon 42 is expanded to press the engagement balloon 44. Thereby, the distal end section 10a can be moved by surely drawing the intestinal wall 40, so that the drawn amount of the intestinal wall 40 can be increased.

Further, in the present embodiment, as in the above described first embodiment, the aspect is preferable in which the internal pressure P2 (step C of Fig. 9, step B of Fig. 11 and step C of Fig. 13) at the time of expanding the first drive balloon 42 or the second drive balloon 46 is controlled to be equal to or greater than the internal pressure P1 of the engagement balloon 44 in the state of engaging with the intestinal wall 40. According to the present aspect, the first drive balloon 42 or the second drive balloon 46 can be expanded to a sufficient size (expansion diameter) without being pressed by the engagement balloon 44, and reduction in the propulsive force can be prevented.

Further, the engagement balloon 44 that has already been practically used can be applied, which leads to high technical feasibility. Further, the engagement force (grip force to the intestinal wall 40) of the engagement balloon 44 which has been already practically used can be applied as it is. Further, when the first drive balloon 42, the engagement balloon 44, and the second drive balloon 46 are separately provided, it is possible to carry out the technical development specifically directed to the respective functions. For example, the first drive balloon 42 and the second drive balloon 46 are not brought into contact with the intestinal wall 40, and hence importance can be placed on the directivity in the material, the shape, the size, and the like, of the first drive balloon 42 and the second drive balloon 46.

Further, the distal end section 10a can be moved forward and backward in the advance direction thereof by suitably combining and performing the forward movement operation and the backward movement operation as described above.

### [Third embodiment]

Next, a third embodiment will be described.

### <Configuration of actuator for moving body in tube>

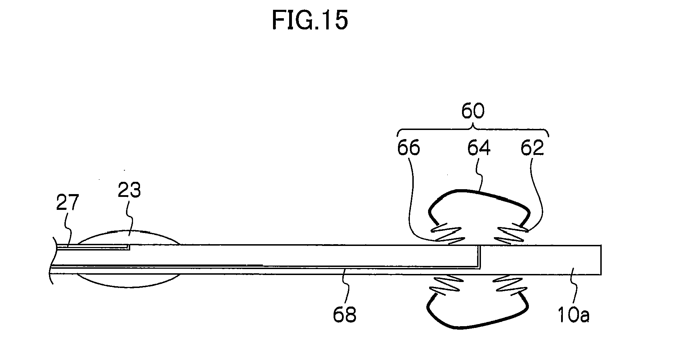

Fig. 15 is an enlarged sectional view showing the distal end section 10a of the insertion section 10 according to a third embodiment. As shown in Fig. 15, in the third embodiment, a propulsion balloon 60 is provided at the distal end section 10a of the insertion section 10. Also, the propulsion balloon 60 is fixed to the insertion section 10. Further, there is also provided the holding balloon 23 which is used to hold the position of the distal end section 10a of the insertion section 10 substantially at the center inside the tube at the time when the propulsion balloon 60 is not brought into contact with the tube wall.

The propulsion balloon 60 is configured by a fixed length section 64, and first and second variable length sections 62 and 66 which are connected to the both ends of the fixed length section 64, respectively.

As a specific configuration of the propulsion balloon 60, it is possible to consider (i) a configuration, the entire part of which is made of natural rubber such as extendable and contractible latex rubber, and in which a shape memory material, artificial muscle, or the like, is stuck only to the portions of the first variable length section 62 and the second variable length section 66, or (ii) a configuration in which only the fixed length section 64 is made of natural rubber such as freely expandable and contractible latex rubber and in which the portions of the first variable length section 62 and the second variable length section 66 are made of a shape memory material, artificial muscle, or the like.

Note that in the case of the configuration, the entire part of which is made of natural rubber such as freely expandable and contractible latex rubber, and in which a shape memory material, artificial muscle, or the like, is stuck only to the portions of the first variable length section 62 and the second variable length section 66, the shape memory material, the artificial muscle, or the like, may be stuck to the entire surface corresponding to the portions of the first variable length section 62 and the second variable length section 66. In that case of the configuration, the shape memory material, the artificial muscle, or the like, formed in a stripe shape may also be stuck to the surface corresponding to the portions of the first variable length section 62 and the second variable length section 66.

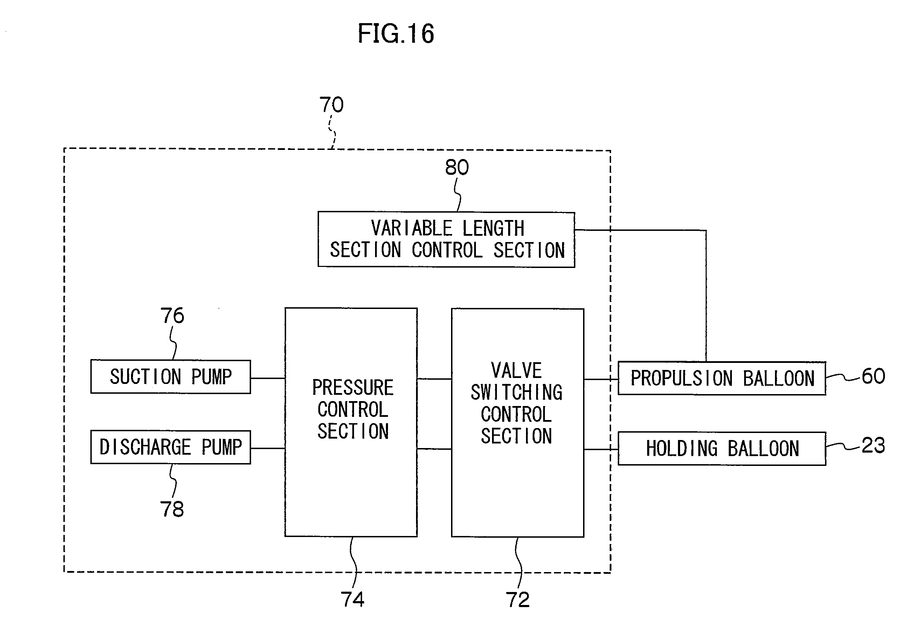

Further, as shown in Fig. 15, a gas supply tube 68 which communicates with the propulsion balloon 60 to supply gas to the propulsion balloon 60, and the gas supply tube 27 which communicates with the holding balloon 23 to supply gas to the holding balloon 23 are provided inside the distal end section 10a. The gas supply tube 68 and the gas supply tube 27 are connected to a balloon control apparatus 70 (see Fig. 16) through the inside of the bending section 10b and the soft section 10c, and the code 14 (see Fig. 1).

Further, Fig. 16 is a block diagram of the balloon control apparatus 70 which controls the pressure of the propulsion balloon 60 and the holding balloon 23. As shown in Fig. 16, the propulsion balloon 60 and the holding balloon 23 are configured such that the internal pressure thereof can be independently adjusted, and are connected to a suction pump 76 and a discharge pump 78 via a valve switching control section 72 and a pressure control section 74.

Further, a variable length section control section 80 which controls the first variable length section 62 and the second variable length section 66 is also provided in the balloon control apparatus 70.

Note that in the flow of the propulsion operation as will be described below, the propulsion operation is performed in such a manner that the opening and closing of valves (not shown) connected to the respective balloons are controlled by the valve switching control section 72, that the suction pump 76 and the discharge pump 78 are controlled by the pressure control section 74, and that the shape memory material, or the like, provided in the first variable length section 62 and the second variable length section 66 is heated or cooled by the variable length section control section 80 so that the first variable length section 62 and the second variable length section 66 are controlled to be contracted or expanded.

### <Flow of propulsion operation>

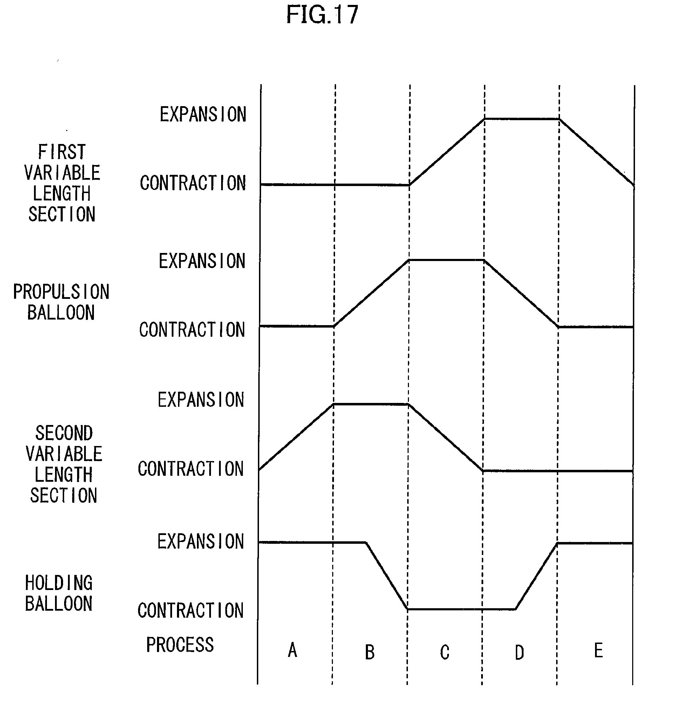

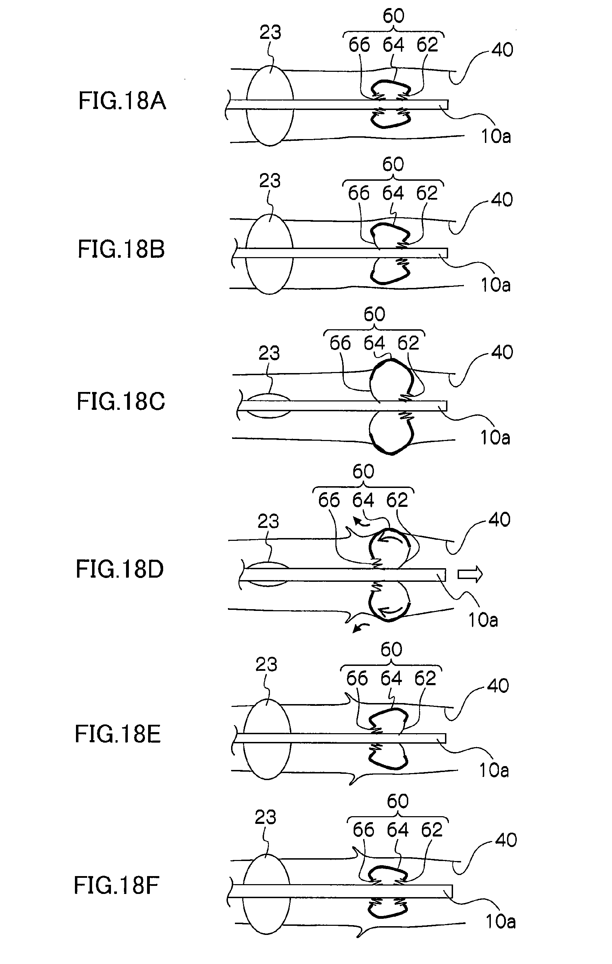

Fig. 17 is a time chart of the propulsion operation in the third embodiment using the actuator for the moving body in tube according to the present invention. Further, Figs. 18A to 18F are schematic sectional views showing states of expansion and contraction of the respective balloons in correspondence with the time chart of the propulsion operation shown in Fig. 17.

First, as shown in Fig. 18A, in the state where the propulsion balloon 60 is contracted, the holding balloon 23 is expanded to engage with the intestinal wall 40.

Next, as shown in Fig. 18B, in the state where the contracted state of the propulsion balloon 60 is maintained, and where the contracted state of the first variable length section 62 is maintained, the shape memory material, or the like, provided at the second variable length section 66 is heated so as to be expanded (process A of Fig. 17).

Next, as shown in Fig. 18C, the propulsion balloon 60 is filled with the gas until the internal pressure thereof reaches a specified value, so that the propulsion balloon 60 is expanded to be brought into close contact with the intestinal wall 40. The gas is discharged from the holding balloon 23 so that the holding balloon 23 is separated from the intestinal wall 40 (process B of Fig. 17).

Here, the specified value of the internal pressure of the propulsion balloon 60 is a pressure value at the time when the sagging of the intestinal wall 40 is eliminated and when the propulsion balloon 60 is brought into close contact with the intestinal wall 40, and also is a pressure value which does not cause breakage of the intestinal wall 40 and which prevents the propulsion balloon 60 from sliding on the intestinal wall 40.

Next, as shown in Fig. 18D, the first variable length section 62 is set in the expanded state from the contracted state by heating the shape memory material, or the like, provided at the first variable length section 62, while the second variable length section 66 is set in the contracted state from the expanded state by cooling the shape memory material, or the like, provided at the second variable length section 66 (process C of Fig. 17).

Thereby, the propulsion balloon 60 generates a backward propulsive force in the advance direction of the distal end section 10a, and hence is able to draw the intestinal wall 40.

Next, as shown in Fig. 18E, the holding balloon 23 is expanded by being filled with the gas, so as to engage with the intestinal wall 40, while the propulsion balloon 60 is contracted by discharging the gas therefrom (process D of Fig. 17).

Next, as shown in Fig. 18F, the first variable length section 62 is set in the contracted state from the expanded state by cooling the shape memory material, or the like, provided at the first variable length section 62 (process E of Fig. 17). Thereby, the states of expansion and contraction of the balloons are returned to the states shown in Fig. 18A.

Thereafter, when the forward movement operation is continued, the processes from process A to process D are repeated.

Note that when the arrangement order of the first variable length section 62 and the second variable length section 66 is reversed and when the second variable length section 66 and the first variable length section 62 are arranged in this order from the front in the advance direction of the distal end section 10a of the insertion section 10, the backward movement operation can be performed.

As described above, in the state where the propulsion balloon 60 (first contraction/expansion member) is made to engage with the intestinal wall 40, the first variable length section 62 (drive device) is set in the expanded state from the contracted state, while the second variable length section 66 (drive device) is set in the contracted state from the expanded state. Thereby, the first variable length section 62 presses the surface of the propulsion balloon 60, or the second variable length section 66 pulls the surface of the propulsion balloon 60, so that the surface of the propulsion balloon 60 is moved.

Here, it is considered that when the propulsion balloon 60 is expanded to come into contact with the intestinal wall 40, a portion of the propulsion balloon 60, which portion fills between the insertion section 10 and the intestinal walls 40 is set as a first portion, and that a portion of the propulsion balloon 60, which portion is in contact with the intestinal wall 40 is set as a second portion. Then, it is also possible to consider that a part of the first portion of the propulsion balloon 60, which part is located on the front side in the advance direction of the distal end section 10a and on the side of the intestinal wall 40, is brought into contact with the intestinal wall 40 and pushed or pulled so as to become the second portion.

Thereby, the propulsive force is transmitted from the surface of the propulsion balloon 60 to the intestinal wall 40, so that the intestinal wall 40 can be drawn.

The above is the description of the propulsion operation of the actuator for the moving body in tube according to the third embodiment.

Note that the propulsion balloon 60 may also be provided at a plurality of places.

### [Fourth embodiment]

### <Configuration of actuator for moving body in tube>

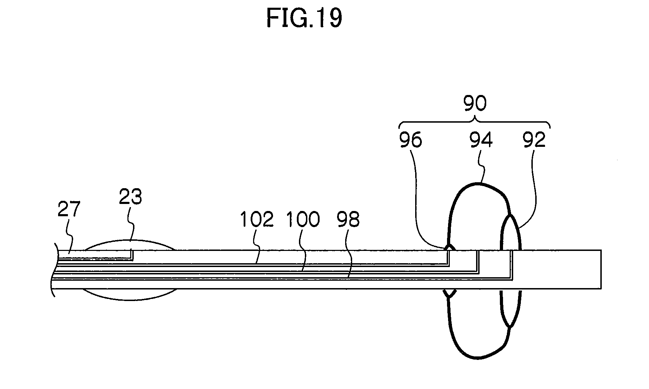

Fig. 19 is an enlarged sectional view showing the distal end section 10a of the insertion section 10 according to a fourth embodiment. As shown in Fig. 19, in the fourth embodiment, a propulsion balloon 90 is provided at the distal end section 10a of the insertion section 10. Also, the propulsion balloon 90 is fixed to the insertion section 10. Further, there is also provided the holding balloon 23 which is used to hold the position of the distal end section 10a of the insertion section 10 substantially at the center inside the tube at the time when the propulsion balloon 90 is not brought into contact with the tube wall.

As shown in Fig. 19, the propulsion balloon 90, the entire part of which is made of natural rubber such as freely expandable and contractible latex rubber, is configured by three pressure chambers of a first sub-gas chamber 92, a main gas chamber 94, and a second sub-gas chamber 96. Further, the first sub-gas chamber 92 and the second sub-gas chamber 96 are arranged on both sides of the main gas chamber 94, respectively.

Further, the three gas chambers are configured such that when the three gas chambers are filled with gas to be expanded most, the volume of the main gas chamber 94 becomes larger than the volume of the first sub-gas chamber 92 and the second sub-gas chamber 96.

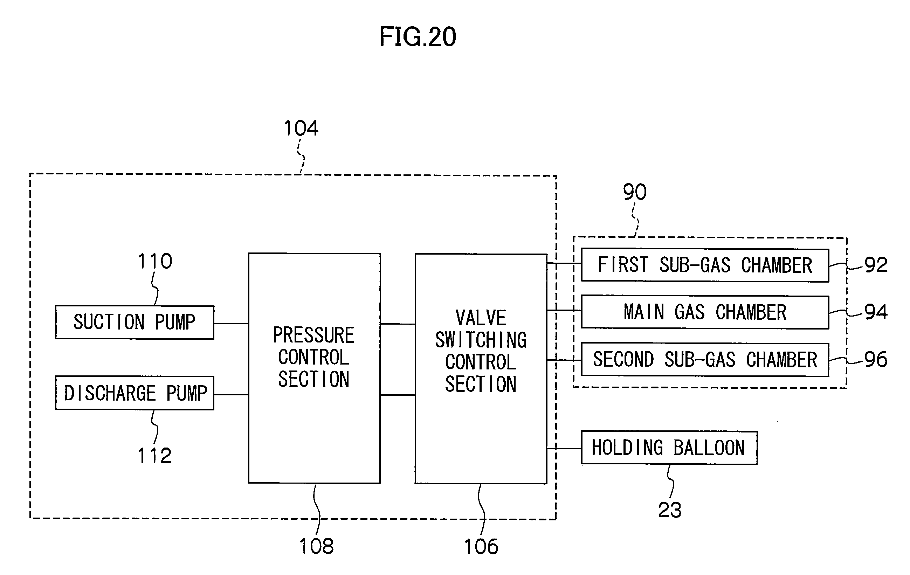

Further, as shown in Fig. 19, a gas supply tube 98 which communicates with the first sub-gas chamber 92 to supply gas to the first sub-gas chamber 92, a gas supply tube 100 which communicates with the main gas chamber 94 to supply gas to the main gas chamber 94, a gas supply tube 102 which communicates with the first sub-gas chamber 96 to supply gas to the first sub-gas chamber 96, and the gas supply tube 27 which communicates with the holding balloon 23 to supply gas to the holding balloon 23, are provided inside the distal end section 10a. The gas supply tube 98, the gas supply tube 100, the gas supply tube 102, and the gas supply tube 27 are connected to a balloon control apparatus 104 (see Fig. 20) through the inside of the bending section 10b and the soft section 10c, and the code 14 (see Fig. 1).

Further, Fig. 20 is a block diagram of the balloon control apparatus 104 which controls the pressure of the propulsion balloon 90 and the holding balloon 23. As shown in Fig. 20, it is configured such that the internal pressure of the propulsion balloon 90 and the holding balloon 23 can be separately independently adjusted. Further, it is configured such that the internal pressure of the three pressure chambers of the first sub-gas chamber 92, the main gas chamber 94, and the second sub-gas chamber 96 of the propulsion balloon 90 can also be separately independently adjusted. The three pressure chambers are connected to a suction pump 110 and a discharge pump 112 via a valve switching control section 106 and a pressure control section 108.

Note that in the flow of the propulsion operation as will be described below, the propulsion operation is performed in such a manner that the opening and closing of valves (not shown) connected to the respective balloons are controlled by the valve switching control section 106, and that the suction pump 110 and the discharge pump 112 are controlled by the pressure control section 108.

### <Flow of propulsion operation>

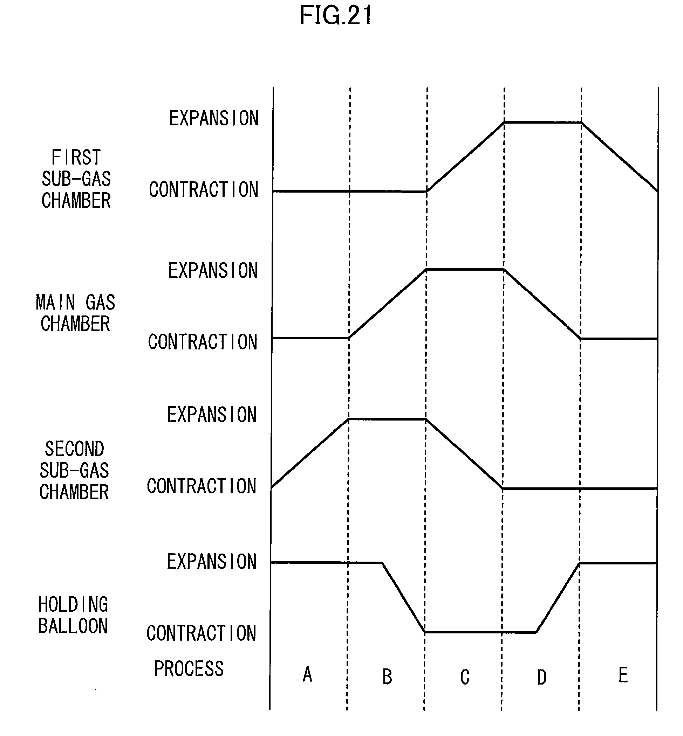

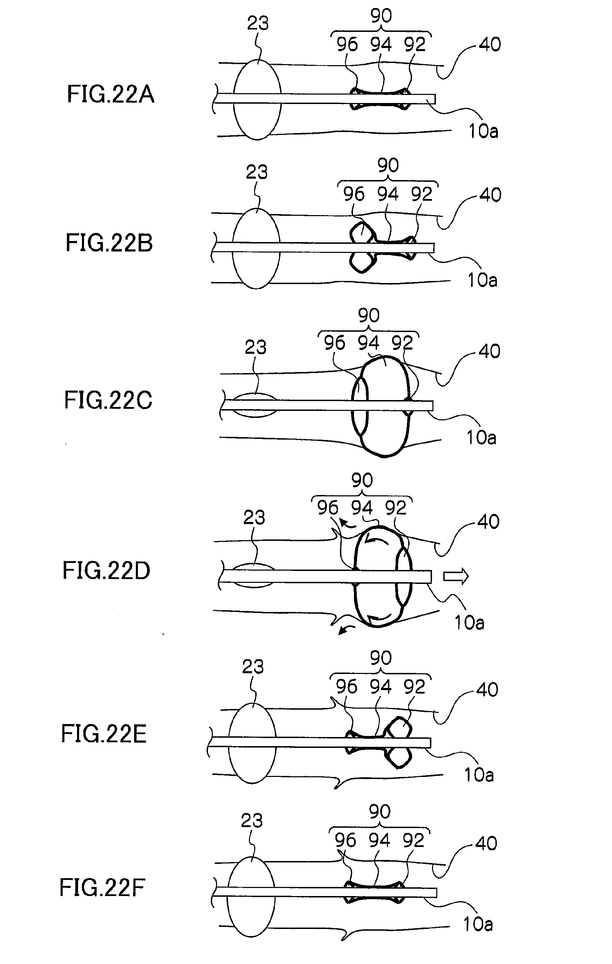

Fig. 21 is a time chart of a specific propulsion operation in the fourth embodiment using the actuator for the moving body in tube according to the present invention. Figs. 22A to 22F are schematic sectional views showing states of expansion and contraction of the respective balloons in the fourth embodiment in correspondence with the time chart shown in Fig. 21.

First, as shown in Fig. 22A, in the state where the first sub-gas chamber 92, the main gas chamber 94, and the second sub-gas chamber 96 are all contracted, the holding balloon 23 is expanded to engage with the intestinal wall 40.

Next, as shown in Fig. 22B, the sub-gas chamber 96 is expanded while the contracted state of the sub-gas chamber 92 and the main gas chamber 94 is maintained (process A of Fig. 21).

Next, as shown in Fig. 22C, the main gas chamber 94 is filled with the gas until the internal pressure thereof reaches a specified value, so that the main gas chamber 94 is expanded to be brought into close contact with the intestinal wall 40. The holding balloon 23 is contracted by discharging the gas therefrom so as to be separated from the intestinal wall 40 (process B of Fig. 21).

Here, the specified value of the internal pressure of the main gas chamber 94 is a pressure value at the time when the sagging of the intestinal wall 40 is eliminated and when the main gas chamber 94 is brought into close contact with the intestinal wall 40, and also is a pressure value which does not cause breakage of the intestinal wall 40 and which prevents the main gas chamber 94 from sliding on the intestinal wall 40.

Next, as shown in Fig. 22D, the first sub-gas chamber 92 is set in the expanded state from the contracted state, while the second sub-gas chamber 96 is set in the contracted state from the expanded state (process C of Fig. 21).

Thereby, the main gas chamber 94 generates backward propulsive force in the advance direction of the distal end section 10a, and hence is able to draw the intestinal wall 40.

Next, as shown in Fig. 22E, the holding balloon 23 is expanded by being filled with the gas, so as to engage with the intestinal wall 40, while the main gas chamber 94 is contracted by discharging the gas therefrom (process D of Fig. 21).

Next, as shown in Fig. 22F, the first sub-gas chamber 92 is set in the contracted state from the expanded state (process E of Fig. 21). Thereby, the states of expansion and contraction of the balloons are returned to the states shown in Fig. 22A.

Thereafter, when the forward movement operation is continued, the processes from process A to process D are repeated.

Note that when the arrangement order of the first sub-gas chamber 92 and the second sub-gas chamber 96 is reversed and when the second sub-gas chamber 96 and the first sub-gas chamber 92 are arranged in this order from the front in the advance direction of the distal end section 10a of the insertion section 10, the backward movement operation can be performed.

As described above, in the state where the main gas chamber 94 of the propulsion balloon 90 (first contraction/expansion member) is made to engage with the intestinal wall 40, the first sub-gas chamber 92 (drive device) is set in the expanded state from the contracted state, while the second sub-gas chamber 96 (drive device) is set in the contracted state from the expanded state. Thereby, the first sub-gas chamber 92 presses the main gas chamber 94, or the second sub-gas chamber 96 pulls the main gas chamber 94, so that the surface of the main gas chamber 94 is moved.

Here, it is considered that when the propulsion balloon 90 is expanded to be brought into contact with the intestinal wall 40, a portion of the propulsion balloon 90, which portion fills between the insertion section 10 and the intestinal walls 40, is set as a first portion, and that a portion of the propulsion balloon 90, which portion is in contact with the intestinal wall 40, is set as a second portion. Then, it is also possible to consider that a part of the first portion of the propulsion balloon 90, which part is located on the front side in the advance direction of the distal end section and on the side of the intestinal wall 40, is brought into contact with the intestinal wall 40 and pushed or pulled so as to become the second portion.

Thereby, the propulsive force is transmitted from the surface of the main gas chamber 94 to the intestinal wall 40, so that the intestinal wall 40 can be drawn.

The above is the description of the propulsion operation of the actuator for the moving body in tube according to the fourth embodiment.

Note that the propulsion balloon 90 may also be provided at a plurality of places.

### <Modification>

Further, in the above described embodiments, examples configured by directly attaching the balloons to the insertion section 10 of the electronic endoscope 1 are described. However, the present invention is not limited to these, and can be applied to an endoscope moving apparatus 120 as shown in Fig. 23.

The endoscope moving apparatus 120 is configured by including: a cylindrical body 122 in which the insertion section 10 is inserted and fixed; the balloons having the specification of any one set of the above described set of the drive balloon 20, the engagement balloon 22, and the holding balloon 23 which are attached at the distal end of the cylindrical body 122, the above described set of the directed drive balloon 28, the engagement balloon 22, and the holding balloon 23, the above described set of the first drive balloon 42, the engagement balloon 44, the second drive balloon 46, and the holding balloon 23, the above described set of the propulsion balloon 60, and the holding balloon 23, and the above described set of the propulsion balloon 90 and the holding balloon 23; and a balloon control apparatus 126 which is connected to a code 124 extended from the from cylindrical body 122, which corresponds to the specification of the balloons attached to the distal end of the cylindrical body 122, and which has the same configuration as that of the above described one of the balloon control apparatus 18, the balloon control apparatus 70, and the balloon control apparatus 104. Fig. 23 is a view showing the drive balloon 20, the engagement balloon 22, and the holding balloon 23 as representatives.

Further, when the insertion section 10 is inserted into the subject, the insertion section 10 is inserted and fixed in the cylindrical body 122, and the insertion section 10 is moved by the control performed by the balloon control apparatus 126 similarly to the above described embodiments.

In the above, the actuator for the moving body in tube according to the present invention, the method for controlling the actuator, and the endoscope are described in detail, but the present invention is not limited to the above described examples. It is obvious that various modification and changes are possible within the scope and spirit of the invention.

## Claims

1. An actuator for a moving body in tube comprising:
a first expansion/contraction member (22, 60, 94) which includes a first portion that fills a space between the moving body in tube and a tube wall at a time of being expanded to come into contact with the tube wall, and a second portion that is brought into contact with the tube wall to generate propulsive force, and a part of which is fixed to the moving body in tube;
a drive device (34, 36, 76, 78) which drives the first expansion/contraction member (20); and
a control section (30, 32, 72, 74, 80) which controls the first expansion/contraction member and the drive device,
wherein the control section performs control so that the first portion of the first expansion/contraction member (22, 60, 94) is made to become the second portion by the drive by the drive device (34, 36, 76, 78) so as to change the relative position between the moving body in tube and the tube wall.

2. The actuator for the moving body in tube according to claim 1,
wherein the drive device is a second expansion/contraction member (20, 28, 92) which is arranged side by side with the first expansion/contraction member (22) in a direction in which the moving body in tube is moved inside the tube, and which is fixed to the moving body in tube, and
wherein the control section (30, 32) performs control so that after the first expansion/contraction member (22) is expanded to engage with the tube wall, the second expansion/contraction member (20, 28, 92) is expanded to press the first expansion/contraction member.

3. The actuator for the moving body in tube according to claim 2,
wherein the control section (30, 32) performs control so that the first expansion/contraction member (22, 94) is pressed by the second expansion/contraction member (20, 28, 92) to thereby cause the tube wall to be drawn.

4. The actuator for the moving body in tube according to one of claims 2 and 3,
wherein the control section (30, 32, 72, 74, 80) performs control so that the tube wall is drawn by feeding the surface of the first expansion/contraction member (22) out.

5. The actuator for the moving body in tube according to one of claims 2 to 4,
wherein the first expansion/contraction member (22, 94), in a state of being expanded to engage with the tube wall, covers at least a part of the contracted second expansion/contraction member (20, 28, 92).

6. The actuator for the moving body in tube according to one of claims 2 to 5,
wherein the second expansion/contraction member (28) has directivity in the direction of deformation.

7. The actuator for the moving body in tube according to one of claims 2 to 6,
wherein at least one of the first expansion/contraction member (22) and the second expansion/contraction member (20, 28) is a balloon.

8. The actuator for the moving body in tube according to one of claims 2 to 7, further comprising
a third expansion/contraction member (46, 96) which is provided at the moving body in tube so as to be arranged side by side with the first expansion/contraction member (22, 94) and the second expansion/contraction member (20, 92) in the direction in which the moving body in tube is moved inside the tube, and which is arranged on the side opposite to the second expansion/contraction member (20, 92) across the first expansion/contraction member (22, 96).

9. The actuator for the moving body in tube according to claim 8,
wherein the control section (30, 32) performs control so that before the first expansion/contraction member (22, 92) is expanded to engage with the tube wall, the third expansion/contraction member (46, 96) is expanded to press the first expansion/contraction member.

10. The actuator for the moving body in tube according to one of claims 8 and 9,
wherein the first expansion/contraction member (22, 94), in the state of being expanded to engage with the tube wall, covers at least a part of the contracted third expansion/contraction member (46, 96).

11. The actuator for the moving body in tube according to one of claims 8 to 10,
wherein the third expansion/contraction member (46) has directivity in the direction of deformation.

12. The actuator for the moving body in tube according to one of claims 2 to 11,
wherein the first expansion/contraction member (22, 94) and the second expansion/contraction member (20, 92) are arranged in this order from the back side in the direction in which the moving body in tube is moved inside the tube.

13. The actuator for the moving body in tube according to one of claims 2 to 12,
wherein a plurality of combinations of the first expansion/contraction member (22, 94) and the second expansion/contraction member (20, 92) are provided at the moving body in tube.

14. The actuator for the moving body in tube according to claim 2,
wherein the control section (30, 32) performs control so that the internal pressure of the second expansion/contraction member (20, 92) becomes equal to or greater than the internal pressure of the first expansion/contraction member (22, 94).

15. The actuator for the moving body in tube according to one of claims 1 to 14,
wherein a holding member (23) which engages with the tube wall to hold the position of the moving body in tube is provided at the moving body in tube.

16. An endoscope (1) comprising one of the actuators for the moving body in tube according to one of claims 1 to 15.

17. A control method of an actuator for a moving body in tube, comprising
performing control to drive a first expansion/contraction member (22, 94), which includes a first portion that fills between the moving body in tube and a tube wall at the time of being expanded to come into contact with the tube wall, and a second portion that is brought into contact with the tube wall to generate propulsive force, and a part of which is fixed to the moving body in tube, so that the relative position between the moving body in tube and the tube wall is changed so as to cause the first portion of the first expansion/contraction member to become the second portion.
